# NEUE EUROPÄISCHE PATENTSCHRIFT

(11) **EP 3 694 350 B2**
(45) Veröffentlichungstag und Bekanntmachung des Hinweises auf die Entscheidung über den Einspruch: **05.03.2025**
(45) Hinweis auf die Patenterteilung: 11.05.2022
(21) Anmeldenummer: 18785947.5
(22) Anmeldetag: 11.10.2018
(51) Int. Cl.: A24F 40/30, A61M 15/06, A61M 11/04, A24F 40/60, A61M 15/00, A61M 16/00

(54) **INHALATOR, INSBESONDERE ELEKTRONISCHES ZIGARETTENPRODUKT**
INHALER, IN PARTICULAR AN ELECTRONIC CIGARETTE PRODUCT
INHALATEUR, NOTAMMENT PRODUIT CIGARETTE ÉLECTRIQUE

(30) Priorität: 13.10.2017 DE 102017123866
(43) Veröffentlichungstag der Anmeldung: 19.08.2020
(73) Patentinhaber: Körber Technologies GmbH, 21033 Hamburg (DE)
(72) Erfinder: NIEBUHR, Gunnar, 22605 Hamburg (DE); SCHMIDT, Rene, 21244 Buchholz i.d.N. (DE); KESSLER, Marc, 22415 Hamburg (DE)
(74) Vertreter: Müller Verweyen
(86) Internationale Anmeldenummer: PCT/EP2018/077728
(87) Internationale Veröffentlichungsnummer: WO 2019/072966

(56) Entgegenhaltungen:
- WO-A1-2014/066730
- WO-A1-2015/076515
- WO-A1-2016/090426
- WO-A1-2017/093535
- WO-A1-2017/153270
- WO-A1-2018/083007
- WO-A2-2011/146174
- WO-A2-2015/038981
- DE-A1- 102016 002 665
- US-A1- 2011 277 757
- US-A1- 2014 060 554
- US-A1- 2014 366 898
- US-A1- 2016 262 454
- US-A1- 2017 231 282
- US-A1- 2017 251 727
- US-A1- 2017 258 140

## Beschreibung

Die vorliegende Erfindung betrifft einen Inhalator, insbesondere ein elektronisches Zigarettenprodukt, umfassend mindestens eine Verdampfereinheit mit mindestens einem elektrischen Verdampfer zum Verdampfen von dem Verdampfer zugeführter Flüssigkeit, und eine elektronische Steuervorrichtung zum Steuern und/oder Regeln der mindestens einen Verdampfereinheit.

In aktuellen elektronischen Zigaretten kommen derzeit zwischen einem und bis zu fünf Verdampferköpfe auf Basis des Docht-Wendels-Prinzips zum Einsatz, die ihr Liquid aus einem gemeinsamen Reservoir beziehen. Der Konsument kann lediglich die Gesamtleistung der Verdampfungsvorrichtung und dadurch die erzeugte Dampfmenge einstellen, weitergehende Einstellungen sind nicht möglich.

Verdampfervorrichtungen, die ein Heizelement in Form einer Heizspule aufweisen, sind beispielsweise aus der US 2014/0366898 A1, WO 2016/090426 A1, US 2017/0251727 A1, WO 2015/038981 A2 und der US 2017/0258140 A1 bekannt.

Die DE 10 2016 002 665 A1 offenbart ein Heizelement, das in einem Strömungskanal angeordnet ist, so dass das Liquid im Strömungskanal verdampft wird.

Die WO 2014/066730 A1 offenbart einen Heizer aus einem Nylonsubstrat und einem Nickelchromdraht.

In der WO 2015/076515 A1 ist das Heizelement durch ein nicht brennbares Metallnetz gebildet.

In der US 2017/0231282 A1 bildet das Heizelement eine Heizkammer, in der das zu verdampfende Medium, beispielsweise Tabak, erhitzt wird.

Die Aufgabe der Erfindung besteht darin, einen Inhalator mit einem höheren Grad an Komfort, einer Vielfalt an Funktionalitäten und / oder einer verbesserten Individualisierbarkeit des Raucherlebnisses bereitzustellen.

Die Erfindung löst diese Aufgabe mit den Merkmalen der unabhängigen Ansprüche. Erfindungsgemäß weist der Inhalator mindestens eine auswechselbare Kartusche auf, die die Verdampfereinheit und einen verbundenen oder verbindbaren Flüssigkeitstank umfasst. Aufgrund der auswechselbaren Kartusche kann der Konsument auf einfache Weise und je nach Wunsch die gewünschte Flüssigkeit in den Inhalator einsetzen und konsumieren, d.h. mit der gewünschten Geschmacksrichtung und/oder dem gewünschten Wirkstoffgehalt und/oder Dampferzeugungspotential.

Gemäß einem ersten Aspekt der Erfindung bildet die Kartusche eine Mehrzahl von Reservoirs zum Speichern einer Mehrzahl von Flüssigkeiten aus. Indem eine Mehrzahl von Flüssigkeiten in dem Inhalator vorgehalten werden, kann ein jeweils gewünschtes Raucherlebnis, durch Auswählen der aktuell zu verdampfenden Flüssigkeit ohne umständlichen Kartuschenwechsel erzielt werden. Wenn beispielsweise ein Reservoir Nikotin und ein Reservoir ein Flavor aufweist, können Nikotinwirkung und Flavorwirkung unabhängig voneinander gesteuert werden, was mit herkömmlichen Einkammersystemen nicht möglich ist. Auch unterschiedliche und veränderbare Aroma-Kombinationen sind möglich.

Zu obigem Zweck weist der Inhalator vorteilhaft ein durch den Konsumenten betätigbares Auswahlelement zum Auswählen eines Reservoirs, aus dem Flüssigkeit verdampft werden soll, aus der Mehrzahl von Reservoirs auf. In einer einfachen und intuitiv zu bedienenden Ausführungsform ist das Auswahlelement ein Drucktaster, wobei durch wiederholtes Betätigen des Drucktasters die Mehrzahl an Reservoirs sequentiell durchlaufen wird. Andere Ausführungsformen des Auswahlelements sind möglich, beispielsweise in Form eines Schiebeschalters.

Vorzugsweise kann der Inhalator in der zuvor geschilderten Variante eine insbesondere optische Anzeige aufweisen, welche den Konsumenten über das ausgewählte Reservoir und/oder über die darin enthaltene Flüssigkeit informiert. Insbesondere kann diese Anzeige eine Charakteristik der ausgewählten Flüssigkeit, insbesondere Geschmacksrichtung, Wirkstoffgehalt und/oder Dampfbildungspotential, anzeigen. Dies kann beispielsweise mittels farblicher Kodierung oder auf andere geeignete Weise geschehen.

In einer vorteilhaften Ausführungsform der Erfindung weist der Inhalator einen Kopf auf, in dem sämtliche Kartuschen und vorteilhaft auch ganz oder teilweise der Energiespeicher des Inhalators angeordnet sind. In dieser Ausführung ist das Gewicht des Inhalators in dem Kopf konzentriert. Der Kopf bildet vorteilhaft eine Handhabe für den Konsumenten aus, so dass der Inhalator durch einhändiges Angreifen am Kopf in der Art einer klassischen Tabakspfeife komfortabel gehalten und zum Mund geführt werden kann. Auf diese Weise ergibt sich eine besonders vorteilhafte Handhabbarkeit des Inhalators.

In der zuvor geschilderten Ausführung weist der Inhalator ein Zugrohr auf, das ein Mundstück des Inhalators mit dem vorgenannten Kopf verbindet. Das Zugrohr dient zur Vergleichmäßigung des Luft/ Dampf/Aerosol-Gemisches, um eine möglichst gleichbleibende Wirkstoff-/Aromagabe zu erzeugen, und ggf. zur Abkühlung heißer Dämpfe oder Gase. Zu diesem Zweck ist die Länge des Zugrohrs vorteilhaft größer als Länge des Kopfes, was zusätzlich die Handhabbarkeit des Inhalators weiter verbessert.

Gemäß einem zweiten Aspekt der Erfindung weist der Inhalator einen reversibel entfernbaren Deckel zum Verschließen einer Öffnung in einem Gehäuse des Inhalators auf, durch die Kartusche in den Inhalator einsetzbar und entnehmbar ist. Der Deckel kann dabei vorteilhaft wiederverwendbar sein. Der Deckel wird vorteilhaft mittels mindestens eines Magnetelements an dem Gehäuse des Inhalators gehalten, was das Abnehmen des Deckels für den Kartuschenwechsel vereinfacht. Es kommen auch andere Verbindungselemente in Betracht, wie beispielsweise Clip-Verbindungen.

In einer bevorzugten Ausführungsform weist der Deckel mindestens eine der Anzahl der in den Inhalator einsetzbaren Kartuschen entsprechende Zahl von Kartuschenaufnahmen auf. Jede Aufnahme weist dann mindestens ein elektrisches Kontaktelement zum Zusammenwirken mit einem entsprechenden elektrischen Kontaktelement der in die Aufnahme einzusetzenden Kartusche auf. Das Einsetzen der Kartuschen in den Deckel kann für den Konsumenten einfacher und komfortabler ausgestaltet werden als das Einsetzen in ein Basisteil des Inhalators. Letztere Variante ist aber nicht ausgeschlossen. In der zuvor geschilderten Ausführungsform weist der Deckel vorteilhaft ein elektrisches Kontaktmittel auf, das mit dem elektrischen Kontaktelement für die Kartusche verbunden und zum Zusammenwirken mit einem entsprechenden, an einem Basisteil des Inhalators angeordneten Kontaktmittel eingerichtet ist, um eine elektrische Verbindung zwischen der mindestens einen Kartusche und dem Basisteil herzustellen.

Ein unvollzähliges Einsetzen von Kartuschen beeinträchtigt die Funktionsfähigkeit des Inhalators vorteilhaft nicht. Jedoch kann ein unvollzähliges Einsetzen von Kartuschen vorteilhaft erkannt werden und je nach Anwendung, beispielsweise bei medizinischer Anwendung, zum Blockieren des Inhalatorbetriebs führen. In ähnlicher Weise kann auch bei vorzeitiger Entleerung einer der Kartuschen verfahren werden. Es wäre auch möglich, durch Drosselung der Entnahme aus einer oder mehrerer Kartuschen, vorzugsweise mit vorheriger Informierung des Benutzers, ein gleichmäßiges Entleeren der Kartuschen sicherzustellen.

Gemäß einem dritten Aspekt der Erfindung weist der Inhalator einen Schalter zum Einstellen unterschiedlicher Betriebsgrößen des Inhalators auf. Insbesondere kann der Schalter zum Einstellen der pro Zug zu erzeugenden Dampfmenge eingerichtet sein, was für den Konsumenten den Vorzug einer einfach und schnell einzustellenden gewünschten Dampfmenge hat. Alternativ oder zusätzlich kann der Schalter zum Einstellen des Zugwiderstands des Inhalators eingerichtet sein. Schließlich kann der Schalter alternativ oder zusätzlich auch als Ein-Aus-Schalter für den Inhalator dienen. Demnach kann der Schalter eine Mehrzahl von Funktionen ausführen, so dass entsprechende separate Schalter zur Ausführung einzelner Funktionen entbehrlich sind, was den Herstellungsaufwand reduziert und die Bedienung des Inhalators vereinfacht.

Gemäß einem vierten Aspekt der Erfindung weist der Inhalator eine induktive Ladeschnittstelle zum induktiven Aufladen des Energiespeichers auf. Eine solche drahtlose Aufladung ist weniger anfällig als eine kabelgebundene Aufladung, beispielsweise über eine USB-Schnittstelle, die aber nicht ausgeschlossen ist.

Gemäß einem fünften Aspekt der Erfindung weist der Inhalator ein Betätigungselement zur Auslösung einer kurzzeitig erhöhten Dampf- und/oder Wirkstoffmenge durch den Konsumenten auf, d.h. einen Verdampfungsschub oder Boost. Dies ermöglicht auf einfache Weise die kurzzeitige Freigabe von besonders viel Dampf für eine vom Konsumenten gewünschte schlagartige Nikotin- und/oder Geschmackswirkung (Kick).

Gemäß einem sechsten Aspekt der Erfindung ist ein Mundstück des Inhalators mittels eines Betätigungselements zwischen einer im Inhalatorgehäuse versenkten Position und einer ausgefahrenen Betriebsposition verstellbar, insbesondere verschiebbar. Dies ermöglicht einen effektiven hygienischen Schutz des Mundstücks in der versenkten Position, wenn der Inhalator nicht in Gebrauch ist. Eine separate aufsetzbare Schutzkappe für das Mundstück, die leicht verloren gehen kann, ist dann entbehrlich.

Besonders vorteilhaft kann durch Verstellen des Mundstücks in die versenkte Position der Inhalator ausgeschaltet und durch Verstellen des Mundstücks in die ausgefahrene Position der Inhalator eingeschaltet werden. Das beschriebene Betätigungselement dient dann gleichzeitig als Ein-Aus-Schalter des Inhalators, so dass ein separater Ein-Aus-Schalter entbehrlich ist.

Weiter vorteilhaft kann durch Verstellen des Mundstücks gleichzeitig der Zugwiderstand des Inhalators veränderbar sein. In diesem Fall kann ein separates Betätigungselement zum Einstellen des Zugwiderstands entbehrlich sein.

Die Erfindung wird im Folgenden anhand bevorzugter Ausführungsformen unter Bezugnahme auf die beigefügten Figuren erläutert. Dabei zeigt
- Fig. 1: eine schematische Darstellung einer E-Zigarette in einer Ausführungsform der Erfindung;
- Fig. 2: eine schematische Darstellung einer weiteren E-Zigarette in einer weiteren Ausführungsform der Erfindung;
- Fig. 3: eine perspektivische Ansicht von schräg oben auf ein pfeifenförmiges elektronisches Zigarettenprodukt;
- Fig. 4: eine Ansicht von unten auf das elektronische Zigarettenprodukt gemäß Figur 3;
- Fig. 5: eine perspektivische Ansicht von schräg unten auf das elektronische Zigarettenprodukt gemäß Figuren 3 und 4;
- Fig. 6: eine perspektivische Ansicht von schräg oben auf einen Teil des elektronischen Zigarettenprodukts gemäß Figuren 3 bis 5;
- Fig. 7: eine perspektivische Ansicht auf ein stabförmiges elektronisches Zigarettenprodukt;
- Fig. 8: eine perspektivische Ansicht auf das Innere des elektronischen Zigarettenprodukts gemäß Figur 7;
- Fig. 9: eine Explosionsdarstellung eines Teils des elektronischen Zigarettenprodukts gemäß Figuren 7 und 8;
- Fig. 10: eine seitliche Ansicht auf ein stabförmiges elektronisches Zigarettenprodukt in einer weiteren Ausführungsform;
- Fig. 11: eine seitliche Ansicht auf das stabförmige elektronisches Zigarettenprodukt gemäß Figur 10 mit eingeschobenem Mundstück; und
- Fig. 12: eine schematische Querschnittsansicht einer beispielhaften Verdampfereinheit für eine E-Zigarette.

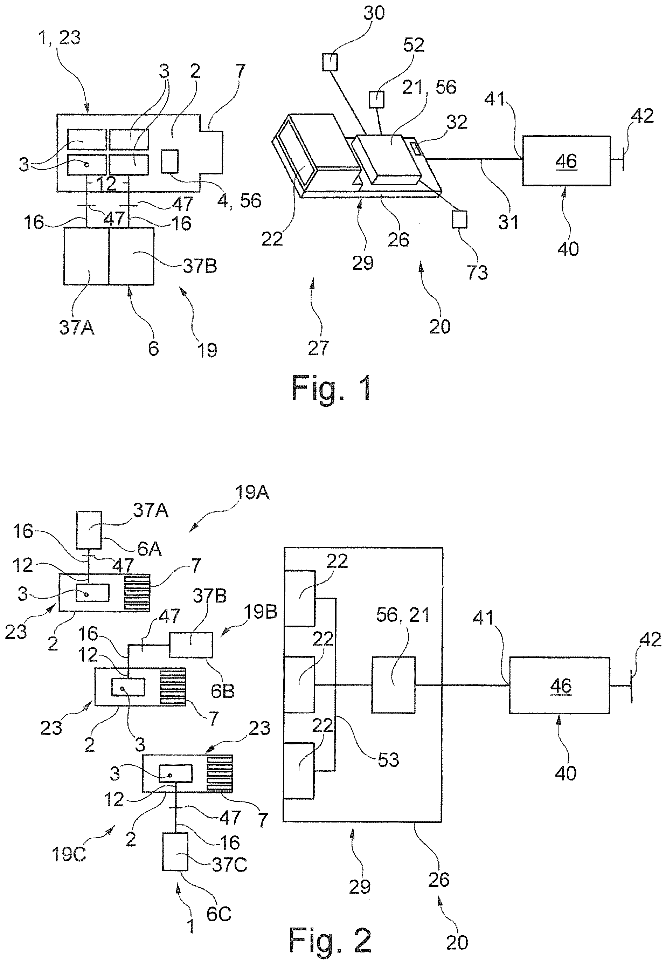

Der Inhalator 27 umfasst eine Verdampfervorrichtung 1 zum Verdampfen von einer oder mehrerer Flüssigkeiten aus einem oder mehreren Flüssigkeitstanks 6, und ein vorteilhaft wiederverwendbares Basisteil 20. Ein vorteilhafter Aufbau des Inhalators 27 und insbesondere der Verdampfervorrichtung 1 ist beispielhaft aus den Figuren 1, 2, 8 und 9 ersichtlich.

Die Verdampfervorrichtung 1 umfasst vorteilhaft eine oder mehrere Verdampfereinheiten 23. Jede Verdampfeinheit 23 umfasst vorteilhaft einen Träger 2, der als Trägerplatte ausgeführt sein kann und mit dem ein Flüssigkeitstank 6 verbunden oder verbindbar ist. Jede Verdampfeinheit 23 weist einen Verdampfer 3 (Figur 2) oder mehrere Verdampfer 3 (Figur 1) auf, die zum Verdampfen von dem oder den Verdampfern 3 aus dem Flüssigkeitstank 6 zugeführter Flüssigkeit dienen und die vorteilhaft auf dem Träger 2 montiert sind. Der vorteilhaft durchgängige, formstabile Träger 2 kann aus einem geeigneten, vorteilhaft nichtleitenden Material bestehen, beispielsweise Keramik oder einem geeigneten Kunststoff, insbesondere PEEK, oder faserverstärktem Kunststoff, beispielsweise PCB-Material.

In der Ausführungsform nach Figur 1 sind die Verdampfer 3 beispielsweise in Matrixform, hier beispielsweise vier Verdampfer 3 in 2x2 Matrixform, angeordnet.

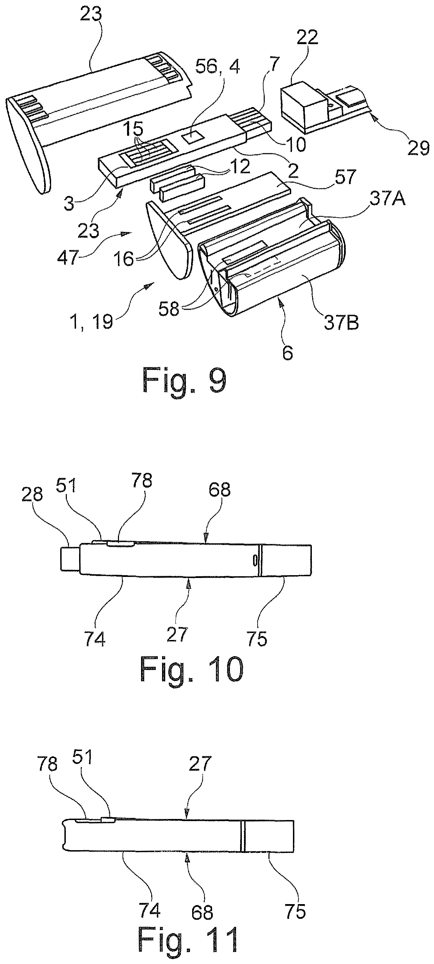

Das dem Verdampfer 3 zugeführte Liquid wird durch den Verdampfer 3 in Dampf/ Aerosol umgesetzt. Der insbesondere elektrische Verdampfer 3 weist mindestens ein, vorzugsweise eine Mehrzahl von elektrischen Widerstands-Heizelementen 15 auf, siehe Figur 9.

Jede Verdampfereinheit 23 ist vorteilhaft in einer baulichen Einheit mit einem Flüssigkeitstank 6 als auswechselbare Kartusche 19 ausgeführt. Jede Kartusche 19 weist vorteilhaft ein Kartuschengehäuse 63 auf, das mindestens teilweise von dem Flüssigkeitstank 6 und/oder mindestens teilweise von der Verdampfereinheit 23 gebildet sein kann. Der mindestens eine Flüssigkeitstank 6 kann wiederauffüllbar sein, so dass die Kartusche 19 ein wiederverwendbares Mehrwegteil ist. Die Kartusche 19 kann alternativ als Einwegteil ausgeführt sein.

Jede Verdampfereinheit 23 bzw. jede Kartusche weist zweckmäßigerweise eine Dampfaustrittsöffnung 64 auf, durch die erzeugter Dampf und/oder Aerosol aus der Verdampfereinheit 23 austreten und dem durch den Inhalator fließenden Luftstrom beigemischt werden kann, um nach Austritt aus dem Inhalator 27 durch die Zugöffnung 24 von dem Konsumenten inhaliert zu werden.

Jede Verdampfereinheit 23 umfasst vorteilhaft eine digitalelektronische Steuereinrichtung 4, beispielsweise eine anwendungsspezifische integrierte Schaltung (ASIC), die vorzugsweise auf dem Träger 2 angeordnet sein kann. Der oder die Verdampfer 3 der Verdampfereinheit 23 können durch die entsprechende elektronische Steuereinrichtung 4 individuell oder in Gruppen angesteuert und mit Strom aus einem Energiespeicher 46 beheizt werden, um an den Heizelementen 15 anliegende Flüssigkeit zu verdampfen.

Die Verdampfereinheit 23 bzw. die Kartusche 19 weist ein elektrisches Kontaktelement 7, hier in Form eines elektrischen Steckers, mit einer Mehrzahl von elektrischen Kontakten 10 auf. Die Kontakte 10 sind mittels elektrischer Leitungen mit der elektronische Steuereinrichtung 4 verbunden, um Sensorsignale, Steuersignale und/oder elektrische Energie an das Basisteil 20 des Inhalators 27 zu übertragen. Die Verdampfereinheit 23 kann Sensoren, beispielsweise einen Temperatursensor zum Messen der Heiztemperatur und/oder einen Drucksensor zum Messen des Strömungsdrucks aufweisen.

Das Basisteil 20 umfasst eine Steuereinheit 29 und eine mit der Steuereinheit 29 verbundene oder zu verbindende Energiespeichereinheit 40. Die Steuereinheit 29 umfasst eine elektronische Steuerung 21 und ein mit dieser verbundenes elektrisches Kontaktelement 22, hier in Form einer zu dem Stecker 7 passenden Steckbuchse. Die Steuereinheit 29 umfasst vorteilhaft des Weiteren eine Nutzerschnittstelle 32, insbesondere eine Drahtlosschnittstelle, beispielsweise eine Bluetooth-Schnittstelle, über welche ein Nutzer den Inhalator 27 mittels eines mobilen Kommunikationsgeräts, beispielsweise eines Smartphones, steuern oder einstellen, und/oder Information von diesem erhalten kann. Die elektronische Steuerung 21 und das elektrische Kontaktelement 22 sind vorteilhaft auf einer gemeinsamen Leiterplatte 26 angeordnet. Die Gesamtheit aus elektronischer Steuereinrichtung 4 und elektronischer Steuerung 21 wird im Rahmen dieser Anmeldung als elektronische Steuervorrichtung 56 des Inhalators 27 bezeichnet.

Der elektrische Stecker 7 und die elektrische Steckbuchse 22 sind einander entsprechend eingerichtet, so dass durch Einstecken des Steckers 7 in die Steckbuchse 22 eine elektrische Verbindung zwischen der Verdampfereinheit 23 und dem Basisteil 20 zur Übertragung von Signalen, Daten und/oder elektrischer Leistung hergestellt wird. In der Verdampfereinheit 23 werden die Versorgungsströme und Signale von dem Stecker 7 an den mindestens einen Verdampfer 3 und/oder an Sensoren weitergeleitet. Vorteilhaft weisen der Stecker 7 und die Steckbuchse 22 jeweils die gleiche Anzahl von elektrischen Kontakten 10 auf.

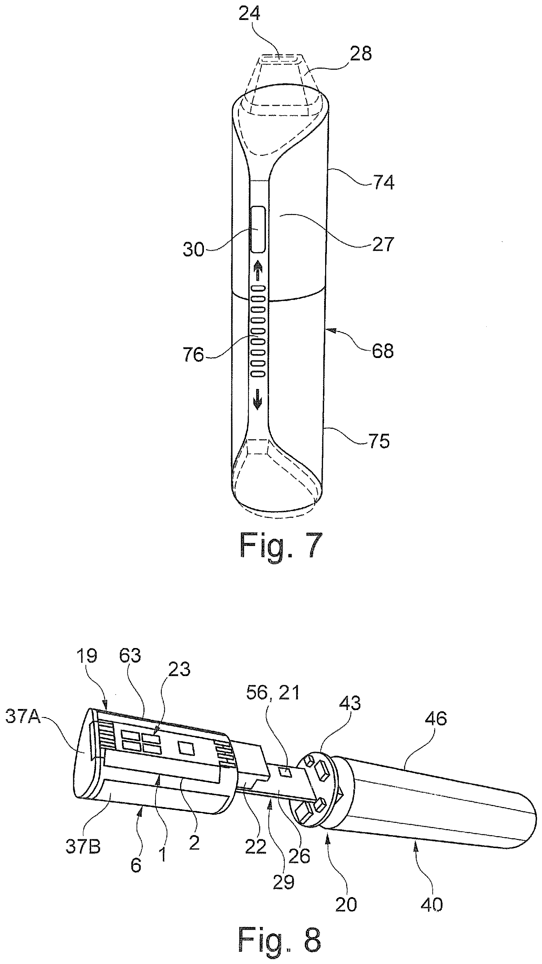

Zum Verbinden der Verdampfereinheit 23 mit dem Basisteil 20 wird in manchen Ausführungsformen, beispielsweise nach Figuren 7 und 8, die Kartusche 19 parallel zur Längsachse des Basisteils 20 in dieses eingeschoben, wodurch der Stecker 7 in die Steckbuche 22 eingeschoben und die elektrische Verbindung hergestellt wird.

Vorteilhaft weist das Basisteil 20 bzw. die Steuereinheit 29 eine der Anzahl der Kartuschen 19 entsprechende Zahl von elektrischen Kontaktelementen 22, hier Steckbuchsen, auf, um den individuellen Austausch einzelner Kartuschen 19 zu ermöglichen. Die Steckbuchsen 22 sind über einen Verteiler 53 mit der elektronischen Steuerung 21 des Basisteils 20 verbunden.

In der elektronischen Steuereinrichtung 4 der Verdampfereinheit 23 ist vorteilhaft eine Kennung bzw. ID (Identifizierungsinformation) der Verdampfereinheit 23 dauerhaft gespeichert. Infolge des Verbindens der Kartusche 19 mit dem Basisteil 20 kann die elektronische Steuerung 21 die Kennung aus der Steuereinrichtung 4 auslesen und eine typgenaue und im Hinblick auf die jeweilige Flüssigkeit optimierte individuelle Steuerung des jeweiligen Verdampfers 3 und/oder der jeweiligen Verdampfereinheit 23 durchführen oder veranlassen, etwa durch Übermittlung von Steuer- und/oder Regelbefehlen an die Steuereinrichtung 4. In der elektronischen Steuerung 21 des Basisteils 20 sind zu diesem Zweck vorzugsweise Steuerdaten für eine Mehrzahl von Kennungen entsprechend einer Mehrzahl von unterschiedlichen Verdampfern 3 bzw. Verdampfertypen und/oder Flüssigkeiten gespeichert, beispielsweise in Form einer Datenbank.

Die Energiespeichereinheit 40 umfasst einen Energiespeicher 46, eine Batterieschnittstelle 41 zum Verbinden der Steuereinheit 29 mit der Energiespeichereinheit 40 über elektrische Leitungen 31, eine Ladeschnittstelle 42 und eine elektronische Schaltung 43 mit Ladeelektronik. Die Steuereinheit 29 wird über die Batterieschnittstelle 41 mit Strom versorgt. Des Weiteren können über die Batterieschnittstelle 41 analoge und/oder digitale Signale zwischen der Energiespeichereinheit 40 und der Steuereinheit 29 übertragen werden. In einer vorteilhaften Ausführungsform umfassen die elektrischen Leitungen 31 einen digitalen Datenbus. Über die elektrische Verbindung 31 zwischen Basisteil 20 und Energiespeichereinheit 40 lassen sich beispielsweise Informationen über den Ladezustand des Energiespeichers 46 oder Diagnosedaten zwischen der Steuereinheit 29 und der Energiespeichereinheit 40 übermitteln. Der Energiespeicher 46 kann eine Einwegbatterie oder ein wiederaufladbarer Akku sein, beispielsweise ein Lithium-Ionen Akku, welcher über die Ladeschnittstelle 42, beispielsweise eine USB-Schnittstelle, oder vorteilhaft drahtlos über eine induktive Ladeschnittstelle, geladen werden kann.

Jede Verdampfereinheit 23 weist vorteilhaft eine standardisierte Flüssigkeitsschnittstelle 47 zur Anbindung des Flüssigkeitstanks 6 an die Verdampfereinheit 23, insbesondere deren Träger 2, auf. Die Flüssigkeitsschnittstelle 47 ist vorteilhaft an der dem Verdampfer 3 entgegengesetzten Seite des Trägers 2 angeordnet. An der Flüssigkeitsschnittstelle 47 wird demnach das Liquid aus dem oder den Reservoirs 37A, 37B bereitgestellt und durch eine vorteilhafte Durchgangsöffnung durch den Träger 2 zu dem oder den Verdampfern 3 geleitet. Die Flüssigkeitsschnittstelle 47 kann beispielsweise mittels eines Dichtelements abgedichtet sein. Der Tank 6 kann für den Konsumenten lösbar oder unlösbar mit der entsprechenden Verdampfereinheit 23 verbunden sein.

Der oder jeder Flüssigkeitstank 6 bildet jeweils ein Flüssigkeitsreservoir 37A, 37B, 37C (Ausführungsform gemäß Figuren 2 bis 6) oder eine Mehrzahl von Flüssigkeitsreservoirs 37A, 37B (Ausführungsformen gemäß Figuren 1, 7 bis 9) aus. Insbesondere kann ein Mehrkammertank 6 zur Ausbildung mehrerer Reservoirs 37A, 37B, ... vorgesehen sein. Beispielhaft ist in den Figur 1 und 7 bis 9 eine Kartusche 19 mit einem Zweikammertank 6 zur Ausbildung zweier Reservoirs 37A, 37B vorgesehen. Selbstverständlich kann ein Flüssigkeitstank 6 auch mehr als zwei Reservoirs 37A, 37B ausbilden. In der Ausführungsform gemäß Figur 1 sind beispielhaft jeweils zwei Verdampfer 3 einem Reservoir 37A, 37B zugeordnet. Selbstverständlich können einem Reservoir 37A, 37B nur ein Verdampfer 3 (siehe Figur 2) oder mehr als zwei Verdampfer 3 zugeordnet sein.

In den Ausführungsformen gemäß Figuren 2 bis 6 sind die Reservoirs 37A, 37B, 37C mittels Einkammertanks 6A, 6B, 6C realisiert. D.h. jeder Tank 6A, 6B, 6C weist nur eine Kammer zur Ausbildung eines Reservoirs 37A, 37B, 37C auf. Im Ausführungsbeispiel gemäß Figur 2 sind beispielsweise drei Kartuschen 19 mit jeweils einer Verdampfereinheit 23 und mit jeweils einem Einkammertank 6A, 6B, 6C vorgesehen. Die Anzahl an Kartuschen 19 bzw. an Tanks 6 kann aber auch eins, zwei oder mehr als drei betragen. Ausführungsformen mit einem einzigen Einkammertank 6 sind umfasst.

Mischformen zwischen Ein- und Mehrkammertanks sind möglich, beispielsweise könnten in den Figuren 2 bis 6 ein Zweikammertank und ein Einkammertank vorgesehen sein.

Jedes Flüssigkeitsreservoir 37A, 37B, ... ist über eine zugeordnete Flüssigkeitszuführung 16 mit einem oder mehreren Verdampfern 3 verbunden, um Flüssigkeit von einer entsprechenden Öffnung 58 des Flüssigkeitstanks 6 zu dem oder den Verdampfern 3 zu transportieren und dort zu verdampfen. Die Flüssigkeitszuführungen 16 können beispielsweise Durchgangsbohrungen in einem Zwischenteil 57 zwischen dem Träger 2 und dem Flüssigkeitstank 6 umfassen, siehe Figur 9.

Zwischen jedem Verdampfer 3 und dem zugeordneten Flüssigkeitsreservoir 37A, 37B, ..., d.h. in der Flüssigkeitszuführung 16, ist vorteilhaft ein Kapillarelement 12 vorgesehen, das Flüssigkeit mittels Kapillarwirkung, beispielsweise mithilfe von Mikrokanälen, von dem Flüssigkeitstank 6 zu dem Verdampfer 3 fördert, um die Benetzung des Verdampfers 3 und die kontinuierliche Nachförderung von Liquid sicherzustellen. Das Kapillarelement 12 kann beispielsweise ein Porenelement mit optimierter Porengröße, ein offenporiges geschäumtes Element, ein Schwammelement und/oder eine Lamellenstruktur umfassen.

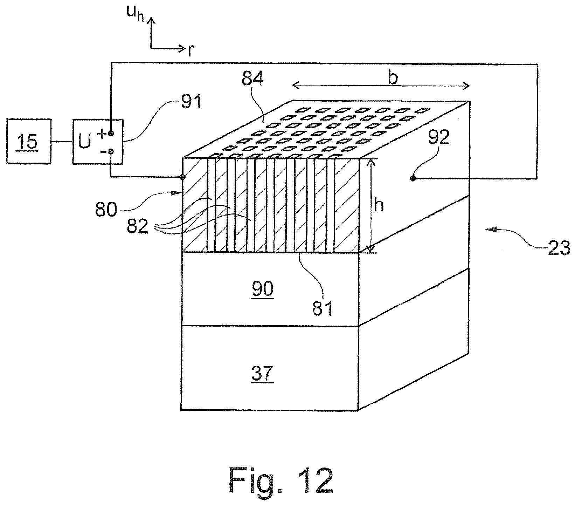

Der oder die Verdampfer 3 können nach Bedarf und Zweckmäßigkeit ausgestaltet sein. Es können beispielsweise Verdampfer 3 mit Leitungs- oder Mikrokanälen zu Einsatz kommen, wie in der DE 10 2016 120 803.5 beschrieben, deren Offenbarungsgehalt insoweit in die vorliegende Anmeldung aufgenommen wird. Diese Variante wird unter Bezugnahme auf Figur 12 noch genauer erläutert. Auch bionische Heizstrukturen, wie bionische Netze, sind für den Verdampfer 3 möglich. Es sind auch Verdampfer 3 mit Heizstrukturen wie in der DE 10 2017 111 119.0 beschrieben möglich, deren Offenbarungsgehalt insoweit in die vorliegende Anmeldung aufgenommen wird.

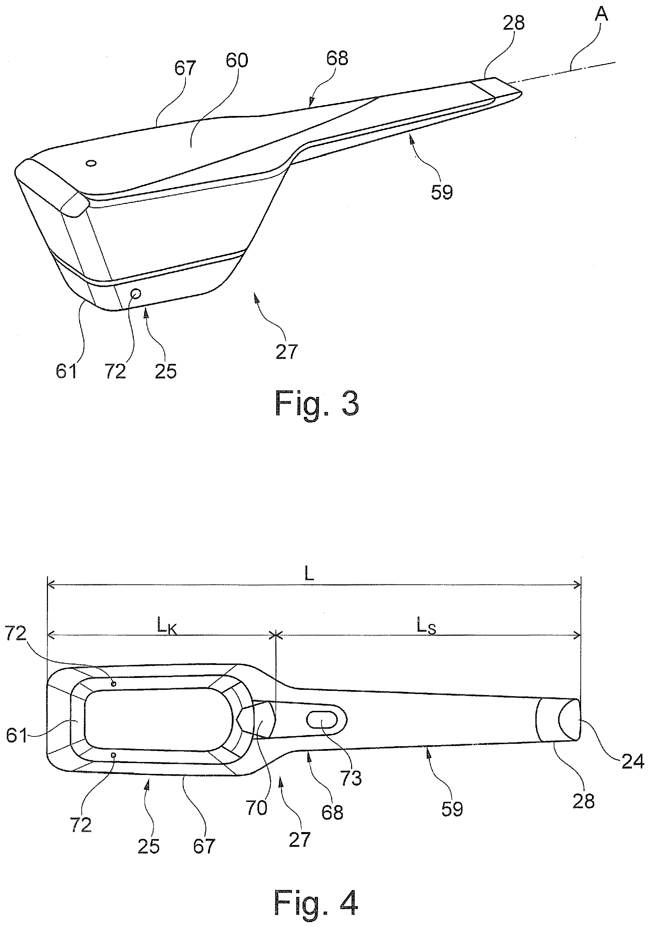

Eine bevorzugte Ausführungsform des Inhalators 27 in der Form einer klassischen Tabakspfeife wird im Folgenden anhand der Figuren 3 bis 6 erläutert. Der Inhalator 27 ist insgesamt länglich und weist am proximalen Ende, d.h. am Zugende, ein Mundstück 28 mit einer Zugöffnung 24 für den Konsumenten auf. Am dem Mundstück 28 entgegengesetzten distalen Ende weist der Inhalator 27 einen Kopf 25 auf. Zwischen dem Mundstück 28 und dem Kopf 25 ist ein Stiel oder Zugrohr 59 vorgesehen, der das Mundstück 28 mit dem Kopf 25 verbindet.

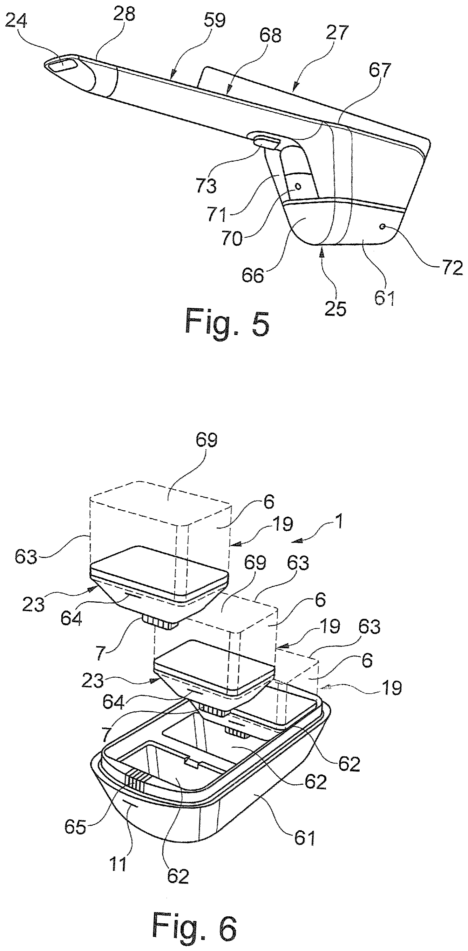

Der Kopf 25 erstreckt sich quer zur Längsachse A des Zugrohrs 59 im Wesentlichen in eine Richtung, nämlich nach unten, wie beispielsweise aus den Figuren 3 und 5 ersichtlich ist. Demnach bilden das Zugrohr 59 und der Kopf 25 eine durchgehende Oberseite 60 des Inhalators. Vorzugsweise verjüngt sich der Kopf 25 nach unten, d.h. mit zunehmender Entfernung von der Rohrachse A.

Die Länge Lk des Kopfes 25 ist vorzugsweise weniger als halb so lang wie die Länge L des Inhalators 27. Die Länge des Zugrohrs 59 ist vorzugsweise mehr als halb so lang wie die Länge L des Inhalators 27. Die Länge Lk des Kopfes 25 ist somit vorteilhaft kleiner als die Länge Ls des Zugrohrs 59, was die Handhabbarkeit des Inhalators 27 verbessert.

Der Inhalator 27 umfasst ein Gehäuse 68, das vorteilhaft zwei vom Konsumenten reversibel miteinander verbindbare Gehäuseteile 61, 67 umfasst, nämlich ein Gehäusegrundteil 67 für den überwiegenden Teil des Inhalators 27, und einen Deckel 61 zum reversiblen Verschließen einer Einsetzöffnung im Kopf 25, der vom Konsumenten abnehmbar ist, um Kartuschen 19 aus dem Inhalator 27 entnehmen und in diesen einsetzen zu können. Der Deckel 61 ist hier als Wanne ausgeführt, die den Kopf 25 an seiner Unterseite abschließt. Der Deckel 61 kann alternativ an der Oberseite 60 des Kopfes 25 oder an einer anderen geeigneten Stelle des Kopfes 25 angeordnet sein.

Der Deckel 61 wird vorteilhaft mittels Magneten an dem Gehäusegrundteil 67 und somit an dem Inhalator 27 gehalten, was für den Konsumenten besonders einfach zu handhaben ist. Alternative Befestigungen, beispielsweise formschlüssige Befestigung etwa mittels Clipselementen, sind möglich.

Die Verdampfervorrichtung 1 ist vorzugsweise vollständig oder im Wesentlichen vollständig in dem Kopf 25 des Inhalators 27 angeordnet. Insbesondere sind die Kartuschen 19 vorzugsweise vollständig in dem Kopf 25 angeordnet. In der Ausführungsform der Figuren 3 bis 6 sind die Kartuschen 19 in den Deckel 61 einsetzbar. Dies ist aus Figur 6 ersichtlich. Der Deckel 61 weist zu diesem Zweck Aufnahmen 62 für die Kartuschen 19 auf, wobei in jede Aufnahme 62 eine Kartusche 19 einsetzbar ist.

In den Aufnahmen 62, insbesondere am Grund derselben, sind zweckmäßigerweise die elektrischen Kontaktelemente 22 zum Zusammenwirken mit den entsprechenden elektrischen Kontaktelementen 7 der Kartuschen 19 angeordnet. Sämtliche elektrischen Kontaktelemente 22 sind über einen Verteiler 53 (siehe Figur 2) mit einem elektrischen Kontaktmittel 65 verbunden. Im Inneren des Kopfes 25 ist ein entsprechendes, nicht gezeigtes elektrisches Kontaktmittel angeordnet. Wenn der Deckel 61 mit dem Kopf 25 verbunden wird, wird das elektrische Kontaktmittel 65 mit dem entsprechenden elektrischen Kontaktmittel im Kopf 25 in Kontakt gebracht, um die die elektrische Verbindung zwischen den Verdampfereinheiten 23 und dem Basisteil 20 herzustellen. Das Kontaktmittel 65 dient somit der Kommunikation zwischen den Kartuschen 19 und dem Basisteil 20.

In einer alternativen Ausführungsform sind die Aufnahmen 62 für die Kartuschen 19 im Kopf 25 statt in dem Deckel 61 vorgesehen, d.h. die Kartuschen 19 sind direkt in den Kopf 25 einsetzbar. In diesem Fall dient der Deckel 61 im Wesentlichen nur zum Verschließen der Einsetzöffnung im Kopf 25. Das Kontaktmittel 65 kann dann entbehrlich sein, der Verteiler 53 ist ggf. in dem Kopf angeordnet.

In dem Gehäuse 68 des Inhalators 27 ist mindestens eine beispielsweise schlitzförmige Lufteintrittsöffnung 66 vorgesehen. Die Lufteintrittsöffnung 66 ist vorteilhaft an dem Kopf 25 und weiter vorteilhaft in dem Deckel 11 angeordnet, siehe Figur 6. Das Zugrohr 59 weist in seinem Inneren einen durchgehenden Luftkanal auf. Wenn der Konsument durch Ziehen an dem Mundstück 28 bzw. der Zugöffnung 24 einen Unterdruck in dem Inhalator 27 erzeugt, tritt Umgebungsluft durch die Lufteintrittsöffnung 11 in den Inhalator ein und durchläuft den Kopf 25 und das Zugrohr 59 bis zum Austritt aus der Zugöffnung 24. Wie zuvor beschrieben wird dieser Luftströmung durch die Verdampfervorrichtung 1 Dampf/Aerosol beigemischt. Das Zugrohr 59 dient zur Vergleichmäßigung des Luft/ Dampf/Aerosol-Gemisches, um eine möglichst gleichbleibende Wirkstoff-/Aromagabe zu erzeugen, und ggf. zur Abkühlung heißer Dämpfe oder Gase.

Im Folgenden wird ohne Beschränkung der Allgemeinheit der Fall betrachtet, dass eine Mehrzahl von Flüssigkeitsreservoirs 37A, 37B, ... für den Inhalator 27 vorgesehen sind. Dabei können Flüssigkeitsreservoirs 37A, 37B, ... unterschiedliche Flüssigkeiten A, B, C und/oder gleiche Flüssigkeiten beinhalten. Die eine oder mehrere Flüssigkeiten in den Reservoirs 37A, 37B, ... umfassen vorteilhaft eine oder mehrere Komponenten aus der Gruppe 1,2-Proplyenglykol, Glycerin, Wasser, mindestens einen Wirkstoff, insbesondere Nikotin, und/oder mindestens einen Aromastoff (Flavour) in gleichen und/oder unterschiedlichen Mischungsverhältnissen umfassen.

Durch den Einsatz mehrerer Reservoirs 37A, 37B, ... und einer Mehrzahl entsprechender Flüssigkeitszuführungen 16 können der oder die Verdampfer 3 unterschiedliche in den Reservoirs 37A, 37B, ... enthaltene Liquids mit unterschiedlicher Zusammensetzung verdampfen. Auf diese Weise kann die Zusammensetzung des von dem oder den Verdampfern 3 erzeugten Dampfs bzw. Aerosols gezielt eingestellt werden. Des Weiteren kann durch gezielte Verdampfung unterschiedlicher Flüssigkeiten die Dampf-/Aerosolzusammensetzung gezielt verändert werden.

Beispielhaft kann der Nikotingehalt vom Konsumenten eingestellt oder verändert werden, wenn Kartuschen 19 mit einer Flüssigkeit A mit Nikotin und einer Flüssigkeit B ohne Nikotin in den Inhalator 27 eingesetzt werden. Des Weiteren kann das Geschmackserlebnis vom Konsumenten eingestellt oder verändert werden, wenn Kartuschen 19 mit einer Flüssigkeit C mit einem Flavour C und einer Flüssigkeit D ohne Flavour C oder mit einem anderen Flavour D in den Inhalator 27 eingesetzt werden.

Die Kartuschen 19 können je nach darin enthaltener Flüssigkeit bzw. Flüssigkeitszusammensetzung für den Konsumenten erkennbar kodiert sein. Beispielsweise können die Kartuschen 19 eine farbliche Kodierung aufweisen, etwa an der dem Steckverbinder 7 entgegengesetzten Oberseite 69 des Kartuschengehäuses 63. Andere optische Kodierungen, beispielsweise mittels grafischen Icons, und/oder eine alphanumerische Beschriftung sind möglich. Aufgrund der Kodierung kann der Konsument einfach und fehlerfrei unterschiedliche Geschmacksrichtungen, Wirkstoffgehalte und/oder Dampferzeugungspotential einzelner Kartuschen 19 erkennen und je nach Wunsch in den Inhalator 27 einsetzen.

Vorteilhaft kann der Konsument einzelne Kartuschen 19 (Figuren 2 bis 6) bzw. einzelne Reservoirs 37A, 37B, ... (Figuren 1, 7 bis 9) auswählen, aus denen Flüssigkeit verdampft werden soll. Zu diesem Zweck weist das Gehäuse 68 des Inhalators, vorteilhaft das Gehäuseteil 67 des Basisteils 20, ein entsprechendes Auswahl- oder Einstellelement 70 auf, siehe Figur 5. Das Auswahlelement 70 kann besonders vorteilhaft an dem Kopf 25, insbesondere in einer der Zugöffnung 24 zugewandten Oberfläche 71 unterhalb des Zugrohrs 59 angeordnet sein.

In einer vorteilhaften Ausgestaltung ist das Auswahl- oder Einstellelement 70 ein Drucktaster. Durch wiederholtes Betätigen des Drucktasters werden dann sämtliche Reservoirs 37A, 37B, ... sequentiell durchlaufen. Im Falle von drei Reservoirs 37A, 37B, 37C würde durch wiederholtes Betätigen des Drucktasters das Reservoir, aus dem Flüssigkeit verdampft werden soll, gemäß folgender Sequenz ausgewählt: 37A, 37B, 37C, 37A, 37B, 37C, 37A, ... usw. Der Drucktaster 70 dient somit zum sequentiellen Durchwechseln sämtlicher Reservoirs 37A, 37B, ... und der darin enthaltenen Flüssigkeiten.

Das Auswahlelement 70 muss kein Drucktaster sein, beispielsweise kann es sich alternativ um einen Schalter, beispielsweise eine Schiebeschalter, vorzugsweise mit einer der Zahl der auswählbaren Reservoirs 37A, 37B, ... entsprechenden Zahl von Schaltstellungen handeln. Andere Ausführungen des Auswahlelements 70 sind möglich.

Infolge der Auswahl eines Reservoirs 37A, 37B, ... durch das Auswahlelement 70 wird (nur) das ausgewählte Reservoir zur Flüssigkeitsversorgung bei der nächsten Verdampfung, d.h. beim nächsten Zug des Konsumenten, herangezogen. Dies kann insbesondere durch individuelle Ansteuerung der dem ausgewählten Reservoir 23 zugeordneten Verdampfungseinheit 23 bzw. Verdampfer 3 geschehen. Zu diesem Zweck sind die den Reservoirs 37A, 37B, ... individuell zugeordneten Verdampfungseinheiten 23 bzw. Verdampfer 3 individuell ansteuerbar.

Vorteilhaft weist der Inhalator 27 eine insbesondere optische Anzeige 72 auf, welche den Konsumenten über das ausgewählte Reservoir 37A, 37B, ... und/oder über die ausgewählte Flüssigkeit informiert. Die optische Anzeige 72 ist vorteilhaft an dem Deckel 61 angeordnet, kann jedoch auch an dem Gehäusegrundteil 67 angeordnet sein. Die optische Anzeige 72 beispielsweise ein oder mehrere LEDs, LCDs oder andere geeignete elektronische Anzeigeelemente umfassen. Im vorliegenden Beispiel umfasst die Anzeige 72 zwei LEDs, nämlich jeweils eine LED auf beiden Seiten des Kopfes 25. Die Anzahl der LEDs kann mehr oder weniger als zwei betragen.

Die optische Anzeige 72 ist insbesondere eingerichtet, um die Charakteristik der ausgewählten Flüssigkeit, insbesondere Geschmacksrichtung, Wirkstoffgehalt und/oder Dampfbildungspotential, anzuzeigen. Dies kann beispielsweise durch veränderbare Farbgebung der optischen Anzeige 72 bzw. der LEDs oder auf andere Weise, beispielsweise Anzeige von grafischen Icons und/oder alphanumerische Anzeige, geschehen.

Vorzugsweise umfasst der Inhalator 27 einen Schalter 73 zum Einstellen unterschiedlicher Betriebsgrößen des Inhalators 27. Insbesondere kann der Schalter 73 zum Einstellen der pro Zug zu erzeugenden Dampfmenge, des Zugwiderstands des Inhalators 27 und/ oder als Ein-Aus-Schalter für den Inhalator 27 dienen. Demnach umfasst der Schalter 73 vorteilhaft eine Nullstellung, in der der Inhalator 27 ausgeschaltet, d.h. die Stromversorgung durch den Energiespeicher 46 unterbrochen ist.

Des Weiteren umfasst der Schalter 73 mindestens eine weitere Stellung, in der der Inhalator 27 eingeschaltet ist. Vorzugsweise sind zwei oder mehr weitere Ein-Stellungen des Schalters 73 vorgesehen, die sich durch unterschiedliche Dampfmengenerzeugung und/oder unterschiedliche Zugwiderstände des Inhalators 27 unterscheiden. Beispielsweise kann eine erste Stellung vorgesehen sein, in der weniger Dampf erzeugt und ein höherer Zugwiderstand des Inhalators 27 eingestellt wird, und eine zweite Stellung, in der mehr Dampf erzeugt und ein niedrigerer Zugwiderstand des Inhalators 27 eingestellt wird. Selbstverständlich können drei oder mehr Ein-Stellungen Schalters 73 vorgesehen sein.

Wenn mit dem Schalter 73, wie beschrieben, gleichzeitig die Dampfmenge und der Zugwiderstand veränderbar ist, wird vorzugsweise mit zunehmender Dampfmenge ein reduzierter Zugwiderstand eingestellt und umgekehrt. Der Schalter 73 kann alternativ nur zur Einstellung der zu erzeugenden Dampfmenge (ohne Beeinflussung des Zugwiderstands), oder nur zur Einstellung des Zugwiderstands des Inhalators 27 (ohne Beeinflussung der zu erzeugenden Dampfmenge) dienen. Des Weiteren kann ein von dem Schalter 73 unabhängiger Ein-Aus-Schalter vorgesehen sein.

Der Zugwiderstand wird generell mittels eines in Figur 1 schematisch dargestellten, den Zugwiderstand beeinflussenden ansteuerbaren Elements 52 in dem Inhalator 27 eingestellt.

Der Schalter 73 kann als Drehschalter oder Schiebeschalter ausgeführt sein. Andere geeignete Ausführungen sind möglich. Der Schalter 73 ist vorteilhaft an der Unterseite des Zugrohrs 59 in geringem Anstand zum Kopf 25 angeordnet. Der Schalter 73 kann an anderer geeigneter Stelle des Inhalators 27 angeordnet sein.

Nach dem oben Gesagten kann der Konsument mithilfe des Auswahlelements 70 und/oder mittels des Schalters 73 flexibel die gewünschte Dampfmenge, Wirkstoffmenge, das gewünschte Aroma und/oder den gewünschten Zugwiderstand einstellen.

Die Ausführungsform gemäß Figuren 7 bis 9 betrifft ein im Wesentlichen stabförmiges elektronisches Zigarettenprodukt 27 mit einer Kartusche 19, die einen Zweikammertank 6 mit zwei Flüssigkeitsspeichern 37A, 37B ausbildet. Die den Flüssigkeitsspeichern 37A, 37B zugeordneten Verdampfer 3 sind unabhängig voneinander ansteuerbar, so dass der Konsument wählen kann, welche Flüssigkeit verdampft werden soll. Auf diese Weise kann der Konsument beispielsweise zwischen einem 100%-igen Nikotinerlebnis (Flüssigkeit A), einem 100%-igen Geschmackserlebnis (Flüssigkeit B) oder einer Mischung aus beidem wählen.

Das Inhalatorgehäuse 68 weist in dieser Ausführungsform zwei Gehäuseteile 74, 75 auf, die für den Konsumenten lösbar miteinander verbunden sind und im getrennten Zustand das reversible Einsetzen bzw. Einstecken der Kartusche 19 in eines der beiden Gehäuseteile 74, 75 ermöglicht, wobei die elektrischen Kontaktelemente 7, 22 miteinander in Wirkverbindung gelangen, um die elektrische Verbindung der Verdampfereinheit 23 in der Kartusche 19 mit dem Basisteil 20 herzustellen.

Außen am Gehäuse 68 und somit für den Konsumenten sichtbar ist vorteilhaft eine Anzeige 76 des Ladestands des Energiespeichers 76 in dem Inhalator und/oder eine Anzeige der restlichen Liquidmenge in dem oder den Reservoirs 37A, 37B, ... des Inhalators 27 vorgesehen.

Zur Auslösung eines Dampfschubs, d.h. einer kurzzeitig erhöhten Dampf- und/oder Wirkstoffmenge (sogenannter Boost), weist das Gehäuse 68 des Inhalators 27 vorteilhaft einen von dem Konsumenten betätigbaren Boost-Betätiger 30, beispielsweise einen Drucktaster, auf. Infolge der Betätigung des Boost-Betätigers 30 steuert die elektronische Steuervorrichtung 56 die Verdampfervorrichtung 1 an, um für einen vordefinierten Zeitraum eine erhöhte Dampf- und/oder Wirkstoffmenge zur erzeugen. Dies kann beispielsweise durch Erhöhung der Verdampfungsleistung der Verdampfer 3 und/oder gleichzeitige Ansteuerung mehrerer oder sämtlicher Verdampfer 3 geschehen. Dabei wird die Leistung der Verdampfer 3 vorteilhaft über Pulsweitenmodulierung eingestellt.

Eine Ausführungsform einer stabförmigen E-Zigarette 27 mit einem verstellbaren Mundstück 28 ist in den Figuren 10 und 11 gezeigt. Das Mundstück 28 ist dabei mittels eines insbesondere mechanischen Betätigungselements 51 vom Konsumenten vorteilhaft entlang der Längsachse der stabförmigen E-Zigarette 27 zwischen den in den Figuren 10 und 11 gezeigten Endpositionen verschiebbar angeordnet. Das Gehäuse 68 weist vorteilhaft eine Nut 78 auf, die parallel zur Längsachse der stabförmigen E-Zigarette 27 angeordnet und in der das Betätigungselement 51 verschiebbar geführt ist.

In der in Figur 10 gezeigten Position ist das Mundstück 29 voll aus dem Inhalatorgehäuse 68 herausgefahrenen, so dass die E-Zigarette 27 betriebsfertig ist.

In der in der in Figur 11 gezeigten Position ist das Mundstück 28 in das Inhalatorgehäuse 68 hineingefahrenen und darin vollständig versenkt. Hierdurch wird ein hygienischer Schutz des Mundstücks 28 im unbetriebenen Zustand der E-Zigarette 27 erreicht. Vorteilhaft wird der Inhalator 27 durch Verstellen in den in Figur 11 gezeigten Zustand mittels des Betätigungselements 51 ausgeschaltet, d.h. die Energiezufuhr von dem Energiespeicher 46 wird unterbrochen. Weiterhin vorteilhaft wird der Inhalator 27 durch Verstellen in den in Figur 10 gezeigten Zustand mittels des Betätigungselements 51 eingeschaltet. Das Betätigungselement 51 dient dann gleichzeitig als Ein-Aus-Schalter, so dass ein separater Ein-Aus-Schalter entbehrlich ist.

Es ist auch vorteilhaft möglich, dass das Betätigungselement 51 Zwischenzustände zwischen denen in Figur 10 und 11 gezeigten Endpositionen einnehmen kann, um auf diese Weise den Zugwiderstand des Inhalators 27 und/oder die Dampfmenge und/oder den Wirkstoffgehalt einstellen zu können. Ein separater Schalter 73 wie in den Figuren 4 und 5 kann dann entbehrlich sein.

Eine vorteilhafte Ausführungsform eines erfindungsgemäßen Verdampfers 3 ist in Fig. 12 gezeigt. Der Verdampfer 3 ist hier durch einen blockförmigen, vorteilhaft monolithischen Heizkörper 80 aus einem elektrisch leitenden Material, vorzugsweise Silizium, dotierte Keramik, Metall-Keramik, Filter-Keramik, Halbleiter, insbesondere Germanium, Graphit, Halbmetall und/oder Metall gebildet. Es ist nicht erforderlich, dass der gesamte Heizkörper 80 aus einem elektrisch leitenden Material besteht. Es kann beispielsweise ausreichen, dass die Oberfläche des Heizkörpers 80 elektrisch leitend, beispielsweise metallisch, beschichtet ist. In diesem Fall muss nicht die gesamte Oberfläche beschichtet sein, beispielsweise können Leiterbahnen auf einem nichtleitenden Grundkörper vorgesehen sein.

Der Heizkörper 80 ist mit einer Mehrzahl von Mikrokanälen 82 versehen, die eine Einlassseite 81 des Heizkörpers 80 mit einer Auslassseite 84 flüssigkeitsleitend verbinden. Die Einlassseite 81 ist beispielsweise über eine Dochtstruktur 90 flüssigkeitsleitend mit dem Flüssigkeitsspeicher 37 verbunden. Die Dochtstruktur 90 dient zur passiven Förderung von Flüssigkeit aus dem Flüssigkeitsspeicher 37 zu dem Heizkörper 80 mittels Kapillarkräften. Die Dochtstruktur 90 im Kontaktbereich 81 zu dem Heizkörper 80 dient dazu, Flüssigkeit gleichmäßig zu verteilen, temperaturbeständig zu sein und mit ihren relativ kleinen Poren und/oder dünnen Kapillaren eine Art Rückschlagventil zu bilden, um unerwünschtes Rückfließen von blasenhaltiger Flüssigkeit aus dem Heizkörper 80 in die Dochtstruktur 90 und/oder in den Flüssigkeitsspeicher 37 zu verhindern.

Anstelle der Dochtstruktur 90 können andere passive und/oder aktive Fördereinrichtungen zum Fördern von Flüssigkeit aus dem Flüssigkeitsspeicher 37 zu dem Heizkörper 80 vorgesehen sein.

Der mittlere Durchmesser der Mikrokanäle 82 liegt vorzugsweise im Bereich zwischen 5 µm und 200 µm, weiter vorzugsweise im Bereich zwischen 30 µm und 150 µm, noch weiter vorzugsweise im Bereich zwischen 50 µm und 100 µm. Aufgrund dieser Abmessungen wird vorteilhaft eine Kapillarwirkung erzeugt, so dass an der Einlassseite 81 in einen Mikrokanal 82 eindringende Flüssigkeit durch den Mikrokanal 82 nach oben steigt, bis der Mikrokanal 82 mit Flüssigkeit gefüllt ist. Das Volumenverhältnis von Mikrokanälen 82 zu Heizkörper 80, das als Porosität des Heizkörpers 80 bezeichnet werden kann, liegt beispielsweise im Bereich zwischen 10% und 50%, vorteilhaft im Bereich zwischen 15% und 40%, noch weiter vorteilhaft im Bereich zwischen 20% und 30%, und beträgt beispielsweise 25%.

Die Kantenlängen der mit Mikrokanälen 82 versehenen Flächen des Heizkörpers 80 liegen beispielsweise im Bereich zwischen 0,5 mm und 3 mm. Die Abmessungen der mit Mikrokanälen 82 versehenen Flächen des Heizkörpers 80 können beispielsweise betragen:
0,95 mm x 1,75 mm; 1,9 mm x 1,75 mm oder 1,9 mm x 0,75 mm. Die Kantenlängen des Heizkörpers 80 können beispielsweise im Bereich zwischen 0,5 mm und 5 mm liegen, vorzugsweise im Bereich zwischen 0,75 mm und 4 mm, weiter vorzugsweise im Bereich zwischen 1 mm und 3 mm liegen. Die Fläche des Heizkörpers 80 (chip size) kann beispielsweise 1 mm x 3 mm oder 2 mm x 3 mm betragen.

Die Breite b des Heizkörpers 80 liegt vorzugsweise im Bereich zwischen 1 mm und 5 mm, weiter vorzugsweise im Bereich zwischen 2 mm und 4 mm, und beträgt beispielsweise 3 mm. Die Höhe h des Heizkörpers 80 liegt vorzugsweise im Bereich zwischen 0,05 mm und 1 mm, weiter vorzugsweise im Bereich zwischen 0,1 mm und 0,75 mm, noch weiter vorzugsweise im Bereich zwischen 0,2 mm und 0,5 mm und beträgt beispielsweise 0,3 mm.

Die Anzahl der Mikrokanäle 82 liegt vorzugsweise im Bereich zwischen vier und 1000. Auf diese Weise lässt sich der Wärmeeintrag von dem Träger in die Mikrokanäle 82 optimieren und eine gesicherte hohe Verdampfungsleistung sowie eine ausreichend große Dampfaustrittsfläche realisieren.

Die Mikrokanäle 82 sind vorteilhaft in Form eines quadratischen, rechteckigen, vieleckigen, runden, ovalen oder anders geformten Arrays angeordnet. Das Array kann in Form einer Matrix mit s Spalten und z Zeilen ausgebildet sein, wobei s vorteilhaft im Bereich zwischen 2 und 50 und weiter vorteilhaft im Bereich zwischen 3 und 30 und/oder z vorteilhaft im Bereich zwischen 2 und 50 und weiter vorteilhaft im Bereich zwischen 3 und 30 liegt. Auf diese Weise lässt sich eine effektive und auf einfache Weise herstellbare Anordnung der Mikrokanäle 82 mit gesichert hoher Verdampfungsleistung realisieren.

Der Querschnitt der Mikrokanäle 82 kann quadratisch, rechteckig, vieleckig, rund, oval oder anders geformt sein, und/oder sich in Längsrichtung abschnittweise ändern, insbesondere vergrößern, verkleinern oder konstant bleiben.

Die Länge eines oder jedes Mikrokanals 82 liegt vorzugsweise im Bereich zwischen 100 µm und 1000 µm, weiter vorzugsweise im Bereich zwischen 150 µm und 750 µm, noch weiter vorzugsweise im Bereich zwischen 180 µm und 500 µm und beträgt beispielsweise 300 µm. Auf diese Weise lässt sich eine optimale Flüssigkeitsaufnahme und Portionsbildung bei ausreichend gutem Wärmeeintrag von dem Heizkörper 80 in die Mikrokanäle 82 realisieren.

Der Abstand zweier Mikrokanäle 82 beträgt vorzugsweise mindestens das 1,3-fache des lichten Durchmessers eines Mikrokanals 82, wobei der Abstand auf die Mittelachsen der beiden Mikrokanäle 82 bezogen ist. Der Abstand kann bevorzugt das 1,5- bis 5-fache, weiter bevorzugt das 2- bis 4-fache des lichten Durchmessers eines Mikrokanals 82 betragen. Auf diese Weise lässt sich ein optimaler Wärmeeintrag von dem Träger in die Mikrokanäle und eine ausreichend stabile Anordnung und Wandstärke der Mikrokanäle realisieren.

Die Verdampfereinheit 20 weist eine vorzugsweise von der Steuervorrichtung 56 steuerbare Heizspannungsquelle 91 auf, die über Elektroden 92 an gegenüberliegenden Seiten des Heizkörpers 80 mit diesem verbunden ist, so dass eine von der Heizspannungsquelle 91 erzeugte elektrische Spannung Uh zu einem Stromfluss durch den Heizkörper 80 führt. Aufgrund des Ohm'schen Widerstands des elektrisch leitenden Heizkörpers 80 führt der Stromfluss zu einer Erhitzung des Heizkörpers 80 und daher zu einer Verdampfung von in den Mikrokanälen 82 enthaltener Flüssigkeit. Der Heizkörper 80 wirkt somit als Verdampfer 3.

Dabei kann die Dauer der einzelnen Verdampfungsschritte bei unterschiedlichen Temperaturen und/oder einem Verdampfen der einzelnen Komponenten der einzelnen Portionen der Flüssigkeit derart kurz gehalten werden und/oder mit einer Ansteuerfrequenz getaktet erfolgen, dass die schrittweise Verdampfung von einem Konsumenten nicht wahrgenommen und trotzdem eine weitgehend homogene, geschmackskonforme, wiederholbar präzise Aerosolbildung gewährleistet werden kann. Insbesondere erfolgt vorteilhaft zunächst ein Verdampfen einer leichter siedenden Komponente der Flüssigkeit in einem ersten Verdampfungsintervall mit einer ersten Temperatur A und anschließend ein Verdampfen einer höher siedenden Komponente der Flüssigkeit in einem zweiten Verdampfungsintervall mit einer zweiten Temperatur B, welche die Temperatur A übersteigt.

Die von der Heizspannungsquelle 91 erzeugte Ansteuerfrequenz des Heizkörpers 80 liegt vorteilhaft im Bereich von 1 Hz bis 50 kHz, bevorzugt im Bereich von 30 Hz bis 30 kHz, noch weiter vorteilhaft im Bereich von 100 Hz bis 25 kHz.

Im Folgenden wird der Ablauf des Verdampfungsvorgangs erläutert.

In einem Ausgangszustand ist die Spannungsquelle 91 für den Heizvorgang ausgeschaltet.

Zum Verdampfen von Flüssigkeit wird die Spannungsquelle 91 für den Heizkörper 80 aktiviert. Die Spannung Uh wird dabei so eingestellt, dass die Verdampfungstemperatur in dem Heizkörper 80 und somit in den Mikrokanälen 82 an das individuelle Verdampfungsverhalten des eingesetzten Flüssigkeitsgemischs angepasst ist. Dies verhindert die Gefahr von lokaler Überhitzung und dadurch Schadstoffentstehung.

Sobald eine Flüssigkeitsmenge verdampft ist, die dem Volumen der Mikrokanäle 82 entspricht oder damit in Zusammenhang steht, wird die Heizspannungsquelle 91 deaktiviert. Da die Liquideigenschaften und -menge vorteilhaft exakt bekannt sind, kann dieser Zeitpunkt sehr genau gesteuert werden. Die Energieaufnahme der Verdampfereinheit 23 lässt sich daher gegenüber bekannten Vorrichtungen reduzieren, da die benötigte Verdampfungsenergie dosierter und damit exakter eingebracht werden kann.

Nach Abschluss des Heizvorgangs sind die Mikrokanäle 82 überwiegend oder vollständig entleert. Die Heizspannung 91 wird dann so lange ausgeschaltet gehalten, bis mittels Nachförderung von Flüssigkeit durch die Dochtstruktur 90 die Mikrokanäle 82 wieder aufgefüllt sind. Sobald dies der Fall ist, kann der nächste Heizzyklus durch Einschalten der Heizspannung 91 begonnen werden.

Der Heizkörper 80 ist vorzugsweise auf der Grundlage von MEMS-Technologie, insbesondere aus Silizium, gefertigt und daher vorteilhaft ein Mikro-Elektro-Mechanisches System.

## Patentansprüche

1. Inhalator (27), insbesondere elektronisches Zigarettenprodukt, umfassend mindestens eine Verdampfereinheit (23) mit mindestens einem elektrischen Verdampfer (3) zum Verdampfen von dem Verdampfer (3) zugeführter Flüssigkeit, und eine elektronische Steuervorrichtung (56) zum Steuern und/oder Regeln der mindestens einen Verdampfereinheit (23), wobei der Inhalator (27) mindestens eine auswechselbare Kartusche (19) aufweist, wobei die Kartusche (19) die Verdampfereinheit (23) und einen verbundenen oder verbindbaren Flüssigkeitstank (6) aufweist, **dadurch gekennzeichnet, dass** der Verdampfer (3) durch einen blockförmigen Heizkörper (80) aus einem elektrisch leitenden Material, vorzugsweise Silizium, gebildet ist, wobei der Heizkörper (80) mit einer Mehrzahl von Mikrokanälen (82) versehen ist, die eine Einlassseite (81) des Heizkörpers (80) mit einer Auslassseite (84) flüssigkeitsleitend verbinden, wobei
- die mindestens eine Kartusche (19) eine Mehrzahl von Reservoirs (37A, 37B, ...) zum Speichern einer Mehrzahl von Flüssigkeiten ausbildet.

2. Inhalator (27), insbesondere elektronisches Zigarettenprodukt, umfassend mindestens eine Verdampfereinheit (23) mit mindestens einem elektrischen Verdampfer (3) zum Verdampfen von dem Verdampfer (3) zugeführter Flüssigkeit, und eine elektronische Steuervorrichtung (56) zum Steuern und/oder Regeln der mindestens einen Verdampfereinheit (23), wobei der Inhalator (27) mindestens eine auswechselbare Kartusche (19) aufweist, wobei die Kartusche (19) die Verdampfereinheit (23) und einen verbundenen oder verbindbaren Flüssigkeitstank (6) aufweist, **dadurch gekennzeichnet, dass** der Verdampfer (3) durch einen blockförmigen Heizkörper (80) aus einem elektrisch leitenden Material, vorzugsweise Silizium, gebildet ist, wobei der Heizkörper (80) mit einer Mehrzahl von Mikrokanälen (82) versehen ist, die eine Einlassseite (81) des Heizkörpers (80) mit einer Auslassseite (84) flüssigkeitsleitend verbinden, wobei
- der Inhalator (27) einen reversibel entfernbaren Deckel (61) zum Verschließen einer Öffnung in einem Gehäuse (68) des Inhalators (27) aufweist, durch die die mindestens eine Kartusche (19) in den Inhalator (27) einsetzbar und entnehmbar ist.

3. Inhalator (27), insbesondere elektronisches Zigarettenprodukt, umfassend mindestens eine Verdampfereinheit (23) mit mindestens einem elektrischen Verdampfer (3) zum Verdampfen von dem Verdampfer (3) zugeführter Flüssigkeit, und eine elektronische Steuervorrichtung (56) zum Steuern und/oder Regeln der mindestens einen Verdampfereinheit (23), wobei der Inhalator (27) mindestens eine auswechselbare Kartusche (19) aufweist, wobei die Kartusche (19) die Verdampfereinheit (23) und einen verbundenen oder verbindbaren Flüssigkeitstank (6) aufweist, **dadurch gekennzeichnet, dass** der Verdampfer (3) durch einen blockförmigen Heizkörper (80) aus einem elektrisch leitenden Material, vorzugsweise Silizium, gebildet ist, wobei der Heizkörper (80) mit einer Mehrzahl von Mikrokanälen (82) versehen ist, die eine Einlassseite (81) des Heizkörpers (80) mit einer Auslassseite (84) flüssigkeitsleitend verbinden, wobei
- der Inhalator (27) einen Schalter (73) zum Einstellen unterschiedlicher Betriebsgrößen des Inhalators (27) aufweist.

4. Inhalator (27), insbesondere elektronisches Zigarettenprodukt, umfassend mindestens eine Verdampfereinheit (23) mit mindestens einem elektrischen Verdampfer (3) zum Verdampfen von dem Verdampfer (3) zugeführter Flüssigkeit, und eine elektronische Steuervorrichtung (56) zum Steuern und/oder Regeln der mindestens einen Verdampfereinheit (23), wobei der Inhalator (27) mindestens eine auswechselbare Kartusche (19) aufweist, wobei die Kartusche (19) die Verdampfereinheit (23) und einen verbundenen oder verbindbaren Flüssigkeitstank (6) aufweist, **dadurch gekennzeichnet, dass** der Verdampfer (3) durch einen blockförmigen Heizkörper (80) aus einem elektrisch leitenden Material, vorzugsweise Silizium, gebildet ist, wobei der Heizkörper (80) mit einer Mehrzahl von Mikrokanälen (82) versehen ist, die eine Einlassseite (81) des Heizkörpers (80) mit einer Auslassseite (84) flüssigkeitsleitend verbinden, wobei
- der Inhalator (27) eine induktive Ladeschnittstelle (42) zum induktiven Aufladen des Energiespeichers (46) aufweist.

5. Inhalator (27), insbesondere elektronisches Zigarettenprodukt, umfassend mindestens eine Verdampfereinheit (23) mit mindestens einem elektrischen Verdampfer (3) zum Verdampfen von dem Verdampfer (3) zugeführter Flüssigkeit, und eine elektronische Steuervorrichtung (56) zum Steuern und/oder Regeln der mindestens einen Verdampfereinheit (23), wobei der Inhalator (27) mindestens eine auswechselbare Kartusche (19) aufweist, wobei die Kartusche (19) die Verdampfereinheit (23) und einen verbundenen oder verbindbaren Flüssigkeitstank (6) aufweist, **dadurch gekennzeichnet, dass** der Verdampfer (3) durch einen blockförmigen Heizkörper (80) aus einem elektrisch leitenden Material, vorzugsweise Silizium, gebildet ist, wobei der Heizkörper (80) mit einer Mehrzahl von Mikrokanälen (82) versehen ist, die eine Einlassseite (81) des Heizkörpers (80) mit einer Auslassseite (84) flüssigkeitsleitend verbinden, wobei
- der Inhalator (27) ein Betätigungselement (30) zur Auslösung einer kurzzeitig erhöhten Dampf- und/oder Wirkstoffmenge aufweist.

6. Inhalator (27), insbesondere elektronisches Zigarettenprodukt, umfassend mindestens eine Verdampfereinheit (23) mit mindestens einem elektrischen Verdampfer (3) zum Verdampfen von dem Verdampfer (3) zugeführter Flüssigkeit, und eine elektronische Steuervorrichtung (56) zum Steuern und/oder Regeln der mindestens einen Verdampfereinheit (23), wobei der Inhalator (27) mindestens eine auswechselbare Kartusche (19) aufweist, wobei die Kartusche (19) die Verdampfereinheit (23) und einen verbundenen oder verbindbaren Flüssigkeitstank (6) aufweist, **dadurch gekennzeichnet, dass** der Verdampfer (3) durch einen blockförmigen Heizkörper (80) aus einem elektrisch leitenden Material, vorzugsweise Silizium, gebildet ist, wobei der Heizkörper (80) mit einer Mehrzahl von Mikrokanälen (82) versehen ist, die eine Einlassseite (81) des Heizkörpers (80) mit einer Auslassseite (84) flüssigkeitsleitend verbinden, wobei
- der Inhalator (27) ein Mundstück (28) umfasst, wobei
- das Mundstück (28) mittels eines Betätigungselements (51) zwischen einer versenkten Position und einer ausgefahrenen Betriebsposition verstellbar, insbesondere verschiebbar ist.

7. Inhalator nach einem der Ansprüche 2 bis 6, **dadurch gekennzeichnet, dass** die mindestens eine Kartusche (19) eine Mehrzahl von Reservoirs (37A, 37B, ...) zum Speichern einer Mehrzahl von Flüssigkeiten ausbildet.

8. Inhalator nach Anspruch 1 oder 7, **dadurch gekennzeichnet, dass** der Inhalator (27) ein betätigbares Auswahlelement (70) zum Auswählen eines Reservoirs, aus dem Flüssigkeit verdampft werden soll, aus der der Mehrzahl von Reservoirs (37A, 37B, ...) aufweist.

9. Inhalator nach Anspruch 8, **dadurch gekennzeichnet, dass** das Auswahlelement (70) ein Drucktaster ist, wobei durch wiederholtes Betätigen des Drucktasters die Mehrzahl an Reservoirs (37A, 37B, ...) sequentiell durchlaufen wird.

10. Inhalator nach Anspruch 8 oder 9, **dadurch gekennzeichnet, dass** der Inhalator (27) eine Anzeige (72) aufweist, welche den Konsumenten über das ausgewählte Reservoir (37A, 37B, ...) und/oder über die darin enthaltene Flüssigkeit informiert.

11. Inhalator nach Anspruch 10, **dadurch gekennzeichnet, dass** die Anzeige (72) eingerichtet ist, um eine Charakteristik der ausgewählten Flüssigkeit, insbesondere Geschmacksrichtung, Wirkstoffgehalt und/oder Dampfbildungspotential, anzuzeigen.

12. Inhalator nach Anspruch 11, **dadurch gekennzeichnet, dass** die Anzeige (72) die Charakteristik der ausgewählten Flüssigkeit mittels einer farblichen Kodierung anzeigt.

13. Inhalator nach einem der vorangehenden Ansprüche, **dadurch gekennzeichnet, dass** der Inhalator (27) einen Kopf (25) aufweist, in dem die oder sämtliche Kartuschen (19) angeordnet ist oder sind.

14. Inhalator nach Anspruch 13, wobei der Inhalator (27) einen Energiespeicher (46) aufweist, **dadurch gekennzeichnet, dass** der Energiespeicher (46) ganz oder teilweise in dem Kopf (25) angeordnet ist

15. Inhalator nach Anspruch 13 oder 14, **dadurch gekennzeichnet, dass** der Inhalator (27) ein Mundstück (28) und ein das Mundstück (28) mit dem Kopf (25) verbindendes Zugrohr (59) aufweist.

16. Inhalator nach Anspruch 15, **dadurch gekennzeichnet, dass** die Länge Ls des Zugrohrs (59) größer ist als die Länge Lk des Kopfes (25).

17. Inhalator nach Anspruch 1 oder nach einem der Ansprüche 3 bis 16, soweit diese nicht auf den Anspruch 2 rückbezogen sind, **dadurch gekennzeichnet, dass** der Inhalator (27) einen reversibel entfernbaren Deckel (61) zum Verschließen einer Öffnung in einem Gehäuse (68) des Inhalators (27) aufweist, durch die die mindestens eine Kartusche (19) in den Inhalator (27) einsetzbar und entnehmbar ist.

18. Inhalator nach Anspruch 2 oder 17, **dadurch gekennzeichnet, dass** der Deckel (61) mittels Magneten an dem Gehäuse (68) des Inhalators (27) gehalten ist.

19. Inhalator nach einem der Ansprüche 2, 17 oder 18, **dadurch gekennzeichnet, dass** der Deckel (61) mindestens eine Aufnahme (62) zum Aufnehmen der mindestens einen Kartusche (19) aufweist, und mindestens ein elektrisches Kontaktelement (22) zum Zusammenwirken mit einem entsprechenden elektrischen Kontaktelement (7) der mindestens einen Kartusche (19).

20. Inhalator nach Anspruch 19, **dadurch gekennzeichnet, dass** der Deckel (61) ein elektrisches Kontaktelement (65) aufweist, das mit dem elektrischen Kontaktelement (22) verbunden und zum Zusammenwirken mit einem entsprechenden, an einem Basisteil (20) des Inhalators (27) angeordneten Kontaktelement eingerichtet ist, um eine elektrische Verbindung zwischen der mindestens einen Kartusche (19) und dem Basisteil (20) herzustellen.

21. Inhalator nach Anspruch 1 oder 2 oder nach einem der Ansprüche 4 bis 20, soweit diese nicht auf den Anspruch 3 rückbezogen sind, **dadurch gekennzeichnet, dass** der Inhalator (27) einen Schalter (73) zum Einstellen unterschiedlicher Betriebsgrößen des Inhalators (27) aufweist.

22. Inhalator nach Anspruch 3 oder 21, **dadurch gekennzeichnet, dass** der Schalter (73) zum Einstellen der pro Zug zu erzeugenden Dampfmenge eingerichtet ist.

23. Inhalator nach einem der Ansprüche 3, 21 oder 22, **dadurch gekennzeichnet, dass** der Schalter (73) zum Einstellen des Zugwiderstands des Inhalators (27) eingerichtet ist, wobei der Zugwiderstand mittels eines den Zugwiderstand beeinflussenden ansteuerbaren Elements (52) in dem Inhalator (27) eingestellt wird.

24. Inhalator nach einem der Ansprüche 3 oder 21 bis 23, **dadurch gekennzeichnet, dass** der Schalter (73) als Ein-Aus-Schalter für den Inhalator (27) dient.

25. Inhalator nach einem der Ansprüche 1 bis 3 oder 5 bis 24, soweit diese nicht auf den Anspruch 4 rückbezogen sind, **dadurch gekennzeichnet, dass** der Inhalator (27) eine induktive Ladeschnittstelle (42) zum induktiven Aufladen des Energiespeichers (46) aufweist.

26. Inhalator nach einem der Ansprüche 1 bis 4 oder 6 bis 25, soweit diese nicht auf den Anspruch 5 rückbezogen sind, **dadurch gekennzeichnet, dass** der Inhalator (27) ein Betätigungselement (30) zur Auslösung einer kurzzeitig erhöhten Dampf- und/oder Wirkstoffmenge aufweist.

27. Inhalator nach einem der Ansprüche 1 bis 5 oder 7 bis 26, soweit diese nicht auf den Anspruch 6 rückbezogen sind, mit einem Mundstück (28), **dadurch gekennzeichnet, dass** das Mundstück (28) mittels eines Betätigungselements (51) zwischen einer versenkten Position und einer ausgefahrenen Betriebsposition verstellbar, insbesondere verschiebbar ist.

28. Inhalator nach Anspruch 6 oder 27, **dadurch gekennzeichnet, dass** durch Verstellen des Mundstücks (28) in die versenkte Position der Inhalator (27) ausgeschaltet und durch Verstellen des Mundstücks (28) in die ausgefahrene Position der Inhalator (27) eingeschaltet wird.

29. Inhalator nach einem der Ansprüche 6, 27 oder 28, **dadurch gekennzeichnet, dass** durch Verstellen des Mundstücks (28) gleichzeitig der Zugwiderstand des Inhalators (27) geändert wird.

## Claims

1. Inhaler (27), in particular electronic cigarette product, comprising at least one evaporator unit (23) with at least one electric evaporator (3) for evaporating liquid supplied to the evaporator (3), and comprising an electronic controller (56) for controlling and/or regulating the at least one evaporator unit (23), wherein the inhaler (27) has at least one replaceable cartridge (19), wherein the cartridge (19) has the evaporator unit (23) and a liquid tank (6) which is connected or can be connected, **characterized in that** the evaporator (3) is formed by a block-shaped heating element (80) made of an electrically conductive material, preferably silicon, wherein the heating element (80) is provided with a plurality of microchannels (82), which connect an inlet side (81) of the heating element (80) to an outlet side (84) in a liquid-conducting manner, wherein
- the at least one cartridge (19) forms a plurality of reservoirs (37A, 37B, ...) for storing a plurality of liquids.

2. Inhaler (27), in particular electronic cigarette product, comprising at least one evaporator unit (23) with at least one electric evaporator (3) for evaporating liquid supplied to the evaporator (3), and comprising an electronic controller (56) for controlling and/or regulating the at least one evaporator unit (23), wherein the inhaler (27) has at least one replaceable cartridge (19), wherein the cartridge (19) has the evaporator unit (23) and a liquid tank (6) which is connected or can be connected, **characterized in that** the evaporator (3) is formed by a block-shaped heating element (80) made of an electrically conductive material, preferably silicon, wherein the heating element (80) is provided with a plurality of microchannels (82), which connect an inlet side (81) of the heating element (80) to an outlet side (84) in a liquid-conducting manner, wherein
- the inhaler (27) has a reversibly removable cover (61) for closing an opening in a housing (68) of the inhaler (27) through which the at least one cartridge (19) can be inserted into the inhaler (27) and removed therefrom.

3. Inhaler (27), in particular electronic cigarette product, comprising at least one evaporator unit (23) with at least one electric evaporator (3) for evaporating liquid supplied to the evaporator (3), and comprising an electronic controller (56) for controlling and/or regulating the at least one evaporator unit (23), wherein the inhaler (27) has at least one replaceable cartridge (19), wherein the cartridge (19) has the evaporator unit (23) and a liquid tank (6) which is connected or can be connected, **characterized in that** the evaporator (3) is formed by a block-shaped heating element (80) made of an electrically conductive material, preferably silicon, wherein the heating element (80) is provided with a plurality of microchannels (82), which connect an inlet side (81) of the heating element (80) to an outlet side (84) in a liquid-conducting manner, wherein
- the inhaler (27) has a switch (73) for setting different operating variables of the inhaler (27).

4. Inhaler (27), in particular electronic cigarette product, comprising at least one evaporator unit (23) with at least one electric evaporator (3) for evaporating liquid supplied to the evaporator (3), and comprising an electronic controller (56) for controlling and/or regulating the at least one evaporator unit (23), wherein the inhaler (27) has at least one replaceable cartridge (19), wherein the cartridge (19) has the evaporator unit (23) and a liquid tank (6) which is connected or can be connected, **characterized in that** the evaporator (3) is formed by a block-shaped heating element (80) made of an electrically conductive material, preferably silicon, wherein the heating element (80) is provided with a plurality of microchannels (82), which connect an inlet side (81) of the heating element (80) to an outlet side (84) in a liquid-conducting manner, wherein
- the inhaler (27) has an inductive charging interface (42) for inductively charging the energy store (46).

5. Inhaler (27), in particular electronic cigarette product, comprising at least one evaporator unit (23) with at least one electric evaporator (3) for evaporating liquid supplied to the evaporator (3), and comprising an electronic controller (56) for controlling and/or regulating the at least one evaporator unit (23), wherein the inhaler (27) has at least one replaceable cartridge (19), wherein the cartridge (19) has the evaporator unit (23) and a liquid tank (6) which is connected or can be connected, **characterized in that** the evaporator (3) is formed by a block-shaped heating element (80) made of an electrically conductive material, preferably silicon, wherein the heating element (80) is provided with a plurality of microchannels (82), which connect an inlet side (81) of the heating element (80) to an outlet side (84) in a liquid-conducting manner, wherein
- the inhaler (27) has an actuating element (30) for triggering a briefly increased amount of vapour and/or active ingredient.

6. Inhaler (27), in particular electronic cigarette product, comprising at least one evaporator unit (23) with at least one electric evaporator (3) for evaporating liquid supplied to the evaporator (3), and comprising an electronic controller (56) for controlling and/or regulating the at least one evaporator unit (23), wherein the inhaler (27) has at least one replaceable cartridge (19), wherein the cartridge (19) has the evaporator unit (23) and a liquid tank (6) which is connected or can be connected, **characterized in that** the evaporator (3) is formed by a block-shaped heating element (80) made of an electrically conductive material, preferably silicon, wherein the heating element (80) is provided with a plurality of microchannels (82), which connect an inlet side (81) of the heating element (80) to an outlet side (84) in a liquid-conducting manner, wherein
- the inhaler (27) has a mouthpiece (28), wherein
- the mouthpiece (28) is adjustable, in particular displaceable, between a lowered position and an extended operating position by means of an actuating element (51).

7. Inhaler according to any one of claims 2 to 6, **characterised in that** the at least one cartridge (19) forms a plurality of reservoirs (37A, 37B, ...) for storing a plurality of liquids

8. Inhaler according to claim 1 or 7, **characterised in that** the inhaler (27) has an actuatable selection element (70) for selecting a reservoir from which liquid is to be evaporated, from the plurality of reservoirs (37A, 37B, ...).

9. Inhaler according to claim 8, **characterised in that** the selection element (70) is a push button, wherein the plurality of reservoirs (37A, 37B, ...) are sequentially run through by repeated actuation of the push button.

10. Inhaler according to claim 8 or claim 9, **characterised in that** the inhaler (27) has an indicator (72) which informs the consumer about the selected reservoir (37A, 37B, ...) and/or about the liquid contained therein.

11. Inhaler according to claim 10, **characterised in that** the indicator (72) is set up to display a characteristic of the selected liquid, in particular flavour, active ingredient content and/or vapour formation potential.

12. Inhaler according to claim 11, **characterised in that** the indicator (72) shows the characteristic of the selected liquid by means of a colour coding.

13. Inhaler according to any one of the preceding claims, **characterised in that** the inhaler (27) has a head (25) in which the cartridge or all of the cartridges (19) is/are arranged.

14. Inhaler according to claim 13, wherein the inhaler (27) has an energy store (46), **characterised in that** the energy store (46) is arranged entirely or in part in the head (25).

15. Inhaler according to claim 13 or claim 14, **characterised in that** the inhaler (27) has a mouthpiece (28) and a puff tube (59) connecting the mouthpiece (28) to the head (25).

16. Inhaler according to claim 15, **characterised in that** the length Ls of the puff tube (59) is greater than the length Lk of the head (25).

17. Inhaler according to claim 1 or any one of claims 3 to 16, as far as these are not referring to claim 2, **characterised in that** the inhaler (27) has a reversibly removable cover (61) for closing an opening in a housing (68) of the inhaler (27) through which the at least one cartridge (19) can be inserted into the inhaler (27) and removed therefrom.

18. Inhaler according to claim 2 or 17, **characterised in that** the cover (61) is held on the housing (68) of the inhaler (27) by means of magnets.

19. Inhaler according to any one of claims 2, 17 or 18, **characterised in that** the cover (61) has at least one receptacle (62) for receiving the at least one cartridge (19), and at least one electrical contact element (22) for interacting with a corresponding electrical contact element (7) of the at least one cartridge (19).

20. Inhaler according to claim 19, **characterised in that** the cover (61) has an electrical contact element (65) which is connected to the electrical contact element (22) and is set up to interact with a corresponding contact element arranged on a base part (20) of the inhaler (27) in order to establish an electrical connection between the at least one cartridge (19) and the base part (20).

21. Inhaler according to claim 1 or 2 or any one of claims 4 to 20, as far as these are not referring to claim 3, **characterised in that** the inhaler (27) has a switch (73) for setting different operating variables of the inhaler (27).

22. Inhaler according to claim 3 or 21, **characterised in that** the switch (73) is set up to adjust the amount of vapour to be generated per puff.

23. Inhaler according to any one of claims 3, 21 or 22, **characterised in that** the switch (73) is set up to adjust the puff resistance of the inhaler (27), wherein the puff resistance is set in the inhaler 27 by means of a controllable element (52) influencing the puff resistance.

24. Inhaler according to any one of claims 3 or 21 to 23, **characterised in that** the switch (73) serves as an on-off switch for the inhaler (27).

25. Inhaler according to any one of claims 1 to 3 or 5 to 24, as far as these are not referring to claim 4, **characterised in that** the inhaler (27) has an inductive charging interface (42) for inductively charging the energy store (46).

26. Inhaler according to any one of claims 1 to 4 or 6 to 25, as far as these are not referring to claim 5, **characterised in that** the inhaler (27) has an actuating element (30) for triggering a briefly increased amount of vapour and/or active ingredient.

27. Inhaler according to any one of claims 1 to 5 or 7 to 26, as far as these are not referring to claim 6, having a mouthpiece (28), **characterised in that** the mouthpiece (28) is adjustable, in particular displaceable, between a lowered position and an extended operating position by means of an actuating element (51).

28. Inhaler according to claim 6 or 27, **characterised in that** the inhaler (27) is switched off by moving the mouthpiece (28) into the lowered position and the inhaler (27) is switched on by moving the mouthpiece (28) into the extended position.

29. Inhaler according to one of claims 6, 27 or claim 28, **characterised in that** by adjusting the mouthpiece (28) the puff resistance of the inhaler (27) is changed at the same time.

## Revendications

1. Inhalateur (27), en particulier produit cigarette électronique, comprenant au moins une unité vaporisateur (23), dotée d'au moins un vaporisateur (3) électrique pour la vaporisation de liquide introduit dans le vaporisateur (3), et un dispositif de commande électronique (56) pour commander et/ou réguler l'au moins une unité vaporisateur (23), l'inhalateur (27) comportant au moins une cartouche (19) remplaçable, la cartouche (19) comportant l'unité vaporisateur (23) et un réservoir de liquide (6) relié ou pouvant être relié, **caractérisé en ce que** le vaporisateur (3) est formé par un corps chauffant (80) en forme de bloc en un matériau électriquement conducteur, de préférence en silicium, le corps chauffant (80) étant pourvu d'une pluralité de microcanaux (82) qui relient un côté entrée (81) du corps chauffant (80) à un côté sortie (84) de manière à permettre un passage de liquide, dans lequel
- l'au moins une cartouche (19) forme une pluralité de réservoirs (37A, 37B, ...) pour stocker une pluralité de liquides.

2. Inhalateur (27), en particulier produit cigarette électronique, comprenant au moins une unité vaporisateur (23), dotée d'au moins un vaporisateur (3) électrique pour la vaporisation de liquide introduit dans le vaporisateur (3), et un dispositif de commande électronique (56) pour commander et/ou réguler l'au moins une unité vaporisateur (23), l'inhalateur (27) comportant au moins une cartouche (19) remplaçable, la cartouche (19) comportant l'unité vaporisateur (23) et un réservoir de liquide (6) relié ou pouvant être relié, **caractérisé en ce que** le vaporisateur (3) est formé par un corps chauffant (80) en forme de bloc en un matériau électriquement conducteur, de préférence en silicium, le corps chauffant (80) étant pourvu d'une pluralité de microcanaux (82) qui relient un côté entrée (81) du corps chauffant (80) à un côté sortie (84) de manière à permettre un passage de liquide, dans lequel
- l'inhalateur (27) comporte un couvercle (61) amovible de manière réversible pour fermer une ouverture dans un boîtier (68) de l'inhalateur (27), par laquelle l'au moins une cartouche (19) peut être insérée dans l'inhalateur (27) et retirée de celui-ci.

3. Inhalateur (27), en particulier produit cigarette électronique, comprenant au moins une unité vaporisateur (23), dotée d'au moins un vaporisateur (3) électrique pour la vaporisation de liquide introduit dans le vaporisateur (3), et un dispositif de commande électronique (56) pour commander et/ou réguler l'au moins une unité vaporisateur (23), l'inhalateur (27) comportant au moins une cartouche (19) remplaçable, la cartouche (19) comportant l'unité vaporisateur (23) et un réservoir de liquide (6) relié ou pouvant être relié, **caractérisé en ce que** le vaporisateur (3) est formé par un corps chauffant (80) en forme de bloc en un matériau électriquement conducteur, de préférence en silicium, le corps chauffant (80) étant pourvu d'une pluralité de microcanaux (82) qui relient un côté entrée (81) du corps chauffant (80) à un côté sortie (84) de manière à permettre un passage de liquide, dans lequel
- l'inhalateur (27) comporte un commutateur (73) pour régler différentes grandeurs de fonctionnement de l'inhalateur (27).

4. Inhalateur (27), en particulier produit cigarette électronique, comprenant au moins une unité vaporisateur (23), dotée d'au moins un vaporisateur (3) électrique pour la vaporisation de liquide introduit dans le vaporisateur (3), et un dispositif de commande électronique (56) pour commander et/ou réguler l'au moins une unité vaporisateur (23), l'inhalateur (27) comportant au moins une cartouche (19) remplaçable, la cartouche (19) comportant l'unité vaporisateur (23) et un réservoir de liquide (6) relié ou pouvant être relié, **caractérisé en ce que** le vaporisateur (3) est formé par un corps chauffant (80) en forme de bloc en un matériau électriquement conducteur, de préférence en silicium, le corps chauffant (80) étant pourvu d'une pluralité de microcanaux (82) qui relient un côté entrée (81) du corps chauffant (80) à un côté sortie (84) de manière à permettre un passage de liquide, dans lequel
- l'inhalateur (27) comporte une interface de charge par induction (42) pour la charge par induction de l'accumulateur d'énergie (46).

5. Inhalateur (27), en particulier produit cigarette électronique, comprenant au moins une unité vaporisateur (23), dotée d'au moins un vaporisateur (3) électrique pour la vaporisation de liquide introduit dans le vaporisateur (3), et un dispositif de commande électronique (56) pour commander et/ou réguler l'au moins une unité vaporisateur (23), l'inhalateur (27) comportant au moins une cartouche (19) remplaçable, la cartouche (19) comportant l'unité vaporisateur (23) et un réservoir de liquide (6) relié ou pouvant être relié, **caractérisé en ce que** le vaporisateur (3) est formé par un corps chauffant (80) en forme de bloc en un matériau électriquement conducteur, de préférence en silicium, le corps chauffant (80) étant pourvu d'une pluralité de microcanaux (82) qui relient un côté entrée (81) du corps chauffant (80) à un côté sortie (84) de manière à permettre un passage de liquide, dans lequel
- l'inhalateur (27) comporte un élément d'actionnement (30) pour déclencher une quantité de vapeur et/ou de substance active momentanément élevée.

6. Inhalateur (27), en particulier produit cigarette électronique, comprenant au moins une unité vaporisateur (23), dotée d'au moins un vaporisateur (3) électrique pour la vaporisation de liquide introduit dans le vaporisateur (3), et un dispositif de commande électronique (56) pour commander et/ou réguler l'au moins une unité vaporisateur (23), l'inhalateur (27) comportant au moins une cartouche (19) remplaçable, la cartouche (19) comportant l'unité vaporisateur (23) et un réservoir de liquide (6) relié ou pouvant être relié, **caractérisé en ce que** le vaporisateur (3) est formé par un corps chauffant (80) en forme de bloc en un matériau électriquement conducteur, de préférence en silicium, le corps chauffant (80) étant pourvu d'une pluralité de microcanaux (82) qui relient un côté entrée (81) du corps chauffant (80) à un côté sortie (84) de manière à permettre un passage de liquide, dans lequel
- l'inhalateur (27) comporte un embout buccal (28), dans lequel
- l'embout buccal (28) peut être réglé, en particulier déplacé, entre une position enfoncée et une position de fonctionnement sortie au moyen d'un élément d'actionnement (51).

7. Inhalateur selon l'une des revendications 2 à 6, **caractérisé en ce que** l'au moins une cartouche (19) forme une pluralité de réservoirs (37A, 37B, ...) pour stocker une pluralité de liquides.

8. Inhalateur selon la revendication 1 ou 7, **caractérisé en ce que** l'inhalateur (27) comporte un élément de sélection (70) actionnable, destiné à sélectionner un réservoir à partir duquel du liquide doit être vaporisé, parmi la pluralité de réservoirs (37A, 37B, ...).

9. Inhalateur selon la revendication 8, **caractérisé en ce que** l'élément de sélection (70) est un bouton-poussoir, la pluralité de réservoirs (37A, 37B, ...) étant parcourue de manière séquentielle par l'actionnement répété du bouton-poussoir.

10. Inhalateur selon la revendication 8 ou 9, **caractérisé en ce que** l'inhalateur (27) comporte un dispositif indicateur (72), qui informe le consommateur sur le réservoir (37A, 37B, ...) sélectionné et/ou sur le liquide contenu dans celui-ci.

11. Inhalateur selon la revendication 10, **caractérisé en ce que** le dispositif indicateur (72) est conçu pour indiquer une caractéristique du liquide sélectionné, en particulier l'arôme, la teneur en substance active et/ou la capacité à former de la vapeur.

12. Inhalateur selon la revendication 11, **caractérisé en ce que** le dispositif indicateur (72) indique la caractéristique du liquide sélectionné au moyen d'un codage couleur.

13. Inhalateur selon l'une des revendications précédentes, **caractérisé en ce que** l'inhalateur (27) comporte une tête (25), dans laquelle la ou toutes les cartouches (19) est ou sont disposée(s).

14. Inhalateur selon la revendication 13, dans lequel l'inhalateur (27) comporte un accumulateur d'énergie (46), **caractérisé en ce que** l'accumulateur d'énergie (46) est disposé entièrement ou en partie dans la tête (25).

15. Inhalateur selon la revendication 13 ou 14, **caractérisé en ce que** l'inhalateur (27) comporte un embout buccal (28) et un tube de tirage (59) reliant l'embout buccal (28) à la tête (25).

16. Inhalateur selon la revendication 15, **caractérisé en ce que** la longueur Ls du tube de tirage (59) est supérieure à la longueur Lk de la tête (25).

17. Inhalateur selon la revendication 1 ou selon l'une des revendications 3 à 16, dans la mesure où elles ne sont pas dépendantes de la revendication 2, **caractérisé en ce que** l'inhalateur (27) comporte un couvercle (61) amovible de manière réversible pour fermer une ouverture dans un boîtier (68) de l'inhalateur (27), par laquelle l'au moins une cartouche (19) peut être insérée dans l'inhalateur (27) et retirée de celui-ci.

18. Inhalateur selon la revendication 2 ou 17, **caractérisé en ce que** le couvercle (61) est maintenu sur le boîtier (68) de l'inhalateur (27) au moyen d'aimants.

19. Inhalateur selon l'une des revendications 2, 17 ou 18, **caractérisé en ce que** le couvercle (61) comporte au moins un logement (62) pour recevoir l'au moins une cartouche (19) et au moins un élément de contact électrique (22) pour coopérer avec un élément de contact électrique (7) correspondant de l'au moins une cartouche (19).

20. Inhalateur selon la revendication 19, **caractérisé en ce que** le couvercle (61) comporte un élément de contact électrique (65), qui est relié à **l'élément** de contact électrique (22) et qui est conçu pour coopérer avec un élément de contact correspondant disposé sur une partie de base (20) de l'inhalateur (27) pour établir une liaison électrique entre l'au moins une cartouche (19) et la partie de base (20).

21. Inhalateur selon la revendication 1 ou 2 ou selon l'une des revendications 4 à 20, dans la mesure où elles ne sont pas dépendantes de la revendication 3, **caractérisé en ce que** l'inhalateur (27) comporte un commutateur (73) pour régler différentes grandeurs de fonctionnement de l'inhalateur (27).

22. Inhalateur selon la revendication 3 ou 21, **caractérisé en ce que** le commutateur (73) est conçu pour régler la quantité de vapeur à produire par bouffée.

23. Inhalateur selon l'une des revendications 3, 21 ou 22, **caractérisé en ce que** le commutateur (73) est conçu pour régler la résistance au tirage de l'inhalateur (27), la résistance au tirage étant réglée au moyen d'un élément (52) dans l'inhalateur (27), qui peut être commandé et qui influence la résistance au tirage.

24. Inhalateur selon l'une des revendications 3 ou 21 à 23, **caractérisé en ce que** le commutateur (73) sert de commutateur marche/arrêt pour l'inhalateur (27).

25. Inhalateur selon l'une des revendications 1 à 3 ou 5 à 24, dans la mesure où elles ne sont pas dépendantes de la revendication 4, **caractérisé en ce que** l'inhalateur (27) comporte une interface de charge par induction (42) pour la charge par induction de l'accumulateur d'énergie (46).

26. Inhalateur selon l'une des revendications 1 à 4 ou 6 à 25, dans la mesure où elles ne sont pas dépendantes de la revendication 5, **caractérisé en ce que** l'inhalateur (27) comporte un élément d'actionnement (30) pour déclencher une quantité de vapeur et/ou de substance active momentanément élevée.

27. Inhalateur selon l'une des revendications 1 à 5 ou 7 à 26, dans la mesure où elles ne sont pas dépendantes de la revendication 6, doté d'un embout buccal (28), **caractérisé en ce que** l'embout buccal (28) peut être réglé, en particulier déplacé, entre une position enfoncée et une position de fonctionnement sortie au moyen d'un élément d'actionnement (51).

28. Inhalateur selon la revendication 6 ou 27, **caractérisé en ce que** l'inhalateur (27) est arrêté par le réglage de l'embout buccal (28) dans la position enfoncée et l'inhalateur (27) est mis en marche par le réglage de l'embout buccal (28) dans la position sortie.

29. Inhalateur selon l'une des revendications 6, 27 ou 28, **caractérisé en ce que** la résistance au tirage de l'inhalateur (27) est modifiée simultanément par le réglage de l'embout buccal (28).
